(19) **Europäisches Patentamt**
**European Patent Office**
**Office européen des brevets**

(11) **EP 1 727 893 B1**

(12) **EUROPEAN PATENT SPECIFICATION**

(45) Date of publication and mention
of the grant of the patent:
**12.12.2012 Bulletin 2012/50**

(51) Int Cl.:
*C12N 5/077* (2010.01)     *C12N 5/0793* (2010.01)

(21) Application number: **05849047.5**

(22) Date of filing: **09.03.2005**

(86) International application number:
**PCT/IB2005/004091**

(87) International publication number:
**WO 2006/048783 (11.05.2006 Gazette 2006/19)**

(54) **AUTOGENIC LIVING SCAFFOLDS AND LIVING TISSUE MATRICES: METHODS AND USES THEREOF**

AUTOGENISCHE LEBENDE GERÜSTE UND LEBENDE GEWEBEMATRIZEN: VERFAHREN UND IHRE VERWENDUNGEN

STRUCTURES DE SUPPORT VIVANTES AUTOGENES ET MATRICES TISSULAIRES VIVANTES: METHODES ET UTILISATIONS ASSOCIEES

(84) Designated Contracting States:
**AT BE BG CH CY CZ DE DK EE ES FI FR GB GR HU IE IS IT LI LT LU MC NL PL PT RO SE SI SK TR**

(30) Priority: **09.03.2004 US 551431 P**

(43) Date of publication of application:
**06.12.2006 Bulletin 2006/49**

(73) Proprietor: **Ahlfors, Jan-Eric W.**
**St. John's (AG)**

(72) Inventor: **Ahlfors, Jan-Eric W.**
**St. John's (AG)**

(74) Representative: **MacLean, Martin Robert et al**
**Mathys & Squire LLP**
**120 Holborn**
**London EC1N 2SQ (GB)**

(56) References cited:
**EP-A- 0 282 746     EP-A- 1 131 410**
**WO-A-02/20729     WO-A-03/018767**

- **MICHEL M ET AL: "Characterization of a new tissue-engineered human skin equivalent with hair." IN VITRO CELLULAR & DEVELOPMENTAL BIOLOGY. ANIMAL. JUN 1999, vol. 35, no. 6, June 1999 (1999-06), pages 318-326, XP008067234 ISSN: 1071-2690**

- **GOSPODAROWICZ D ET AL: "STRUCTURAL CHARACTERIZATION AND BIOLOGICAL FUNCTIONS OF FIBROBLAST GROWTH FACTOR" ENDOCRINE REVIEWS, BALTIMORE, MD, US, vol. 8, no. 2, 1987, pages 95-114, XP009003095**

- **PAINO C L ET AL: "Induction of axon growth into Schwann cell implants grafted into lesioned adult rat spinal cord." EXPERIMENTAL NEUROLOGY. NOV 1991, vol. 114, no. 2, November 1991 (1991-11), pages 254-257, XP002391631 ISSN: 0014-4886**

- **MURAKAMI TAKESHI ET AL: "Transplanted neuronal progenitor cells in a peripheral nerve gap promote nerve repair." BRAIN RESEARCH. 6 JUN 2003, vol. 974, no. 1-2, 6 June 2003 (2003-06-06), pages 17-24, XP002392826 ISSN: 0006-8993**

- **AHLFORS, JAN-ERIC: "A Comparative Analysis of the Biomechanics and Biochemistry of Cell-Derived and Cell-Remodeled Matrices: Implications for Wound Healing and Regenerative Medicine"[Online] 24 April 2004 (2004-04-24), XP002391632 thesis presentation Retrieved from the Internet: URL:HTTP://WWW.WPI.EDU/PUBS/ETD/AVAILABLE/ ETD-0503104-172949/>**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

EP 1 727 893 B1

- L'HEUREUX N ET AL: "A completely biological tissue-engineered human blood vessel.", THE FASEB JOURNAL : OFFICIAL PUBLICATION OF THE FEDERATION OF AMERICAN SOCIETIES FOR EXPERIMENTAL BIOLOGY JAN 1998 LNKD- PUBMED:9438410, vol. 12, no. 1, January 1998 (1998-01), pages 47-56, XP002942845, ISSN: 0892-6638

- ISHIKAWA O ET AL: "Morphological and biochemical analyses on fibroblasts and self-produced collagens in a novel three-dimensional culture.", THE BRITISH JOURNAL OF DERMATOLOGY JAN 1997 LNKD- PUBMED: 9039287, vol. 136, no. 1, January 1997 (1997-01), pages 6-11, ISSN: 0007-0963

## Description

### Technical Field

The present invention relates to "living scaffolds" particularly "autogenic living scaffolds" (ALS), that comprise suitable living cells and the extracellular matrices these living cells produce. The invention also relates to the use of such autogenic living scaffolds as templates and supporting structures for growth of the same cells and tissue or the growth of different cells and different tissue

### Background Art

In the United States, millions of people are affected by tissue loss every year. Current treatments include tissue transfer from a healthy site in the same or another individual, use of medical devices to support the function of the lost tissue, or pharmacologic supplementation of the metabolic products of the lost tissue. Problems with these current treatments include potential tissue complications and imperfect matches including the possible dependence on immunosuppressants, limited durability of the mechanical devices, and the inconvenience and complexity of pharmacologic supplementation. Current approaches for developing living tissue substitutes make use of a "scaffold" that serves as a physical support and template for cell attachment and tissue development. These scaffolds are ideally designed to resemble, both in structure and composition, the extracellular matrix that the cells are exposed to *in vivo,* in order to simulate the *in vivo* conditions. An early and widely used natural scaffold is made of the extracellular matrix protein collagen, while more recently, mechanically stronger artificial scaffolds made of substances such as poly-glycolic acid (PGA) and poly-lactic acid (PLA) have been used.

Some cell-scaffold compositions have multiple layers of biocompatible materials including extracellular matrix materials such as collagen, fibril-forming collagen, Matrix Gla protein, osteocalcin, or other biocompatible materials including marine coral, coralline hydroxyapatite ceramic, and mixtures thereof, and some such scaffolds have been seeded with cells, and then placed within a bioreactor having a means for mechanically stimulating the cells at distinct frequencies (see U.S. Patent Application No. 0040005297 to P. R. Connelly et al., filed July 8, 2002, published January 8, 2004).

In addition, living tissue equivalents (LTEs), notably cell-seeded collagen and fibrin gels, have been used extensively as *in vitro* wound-healing models as well as systems for studying tissue remodeling. More recently, LTEs have begun to gain considerable attention as replacements for lost or damaged connective tissue (e.g., Apligraf™ from Organogenesis, Inc.). LTEs have several advantages over synthetic alternatives including being a natural cell substrate, allowing cellularity to be achieved directly, and being conducive to cell spreading and extracellular matrix (ECM) formation. LTEs are made by mixing cells with a soluble biopolymer solution (e.g., collagen, fibrin, and/or proteoglycans). The cells invade, rearrange and partially degrade the biopolymer scaffold over the next few days as well as synthesize new proteins throughout the culture period. However, LTEs generally lack the physical properties necessary to resist *in vivo* mechanical forces, and are not true "living tissues".

Over the last two decades, LTEs that are completely cell-derived have been developed. However, to date they have been very thin and taken a long time to grow, generally on the order of months, whereas collagen gels and fibrin gels can be developed in only a few days. There is a need for completely biological cell-derived LTEs, and living scaffolds for use in wound repair and tissue regeneration *in vitro* and *in vivo.*

WO 00/29553 (= EP1131410) describes bioengineered tissue constructs and methods for producing and using them.

### Summary of the Invention

The present invention provides a living three-dimensional construct, together with corresponding methods for using said construct, as defined in the claims. The present specification provides a strong, thick, cell-produced living tissue equivalent (LTE), comprising cells and ECM produced by these cells (this ECM is called cell-produced matrix or cell-derived matrix (CDM)), that can be developed in only three weeks for use in creating strong and completely biological soft connective tissue substitutes and for examining wound-healing and tissue development *in vitro.* The biomechanics and corresponding biochemical composition of cell-produced and cell-remodeled matrices are also provided, as are chemically-defined media permissive to the self-production of extracellular matrix (ECM) by cells.

Other embodiments disclose placing fibroblasts in conditions that are conducive to the rapid production of extracellular matrix without an exogenous scaffold, which results in a significantly stronger and thicker 3-D construct than can be obtained with cell-remodeled matrices, such as fibroblast-populated collagen and fibrin gels.

Thus, embodiments as described herein provide for the use of autogenic living scaffolds, cell produced matrices (also referred to as cell-derived matrices (CDMs), and living tissue matrices (LTMs) made entirely of living cells and the extracellular matrices they produce *in vitro,* to promote differentiation, dedifferentiation and/or transdifferentiation of cells and formation of tissue *in vitro* and *in vivo,* while at the same time promoting cell growth, proliferation, migration and/or acquisition of in vivo-like morphology, none of which has been reported to date.

Embodiments as described herein provide an autogenic living scaffold (ALS) or living tissue matrix (LTM) that is a cell-produced scaffold which provides mechanical, nutritional and/or developmental support for cells, tissues, organs, or combinations thereof. The cell-produced autogenic living scaffold as herein disclosed is smart, such that it is capable of adjusting to its environment, and it is living, whereby it is biologically active and all components except seeded cells and tissue are naturally formed by the scaffold system itself, making the scaffold autogenic, or self-produced.

The Autogenic Living Scaffolds as disclosed herein are made by and comprise living cells and the extracellular matrix (ECM) that the living cells produce. The living cells may be genetically engineered or otherwise modified. The ALS serves as a blueprint, supporting structure, backbone, or scaffold for the same or other cell lines or types. The ALS may also provide proper or supporting mechanical and chemical environments, signals, or stimuli to other cells, to the cells that produce the ALS, to surrounding tissue at an implantation site, to a wound, or for in vitro generation and regeneration of cells, tissue and organs. The ALS may also provide other cells with nutrients, growth factors, and other necessary or useful components, may take in or serve as buffers for certain substances in the environment, and have the potential to adapt to new environments.

The cells of an Autogenic Living Scaffold may also be used to produce tissue and/or organs such as cardiac muscle, when seeded with cells or tissue of interest. For example, liver tissue may be produced from an ALS that is seeded with hepatocytes; and kidney, pancreas, spinal cord, and other organs and tissues of the body may also be produced by seeding the ALS with the desired cell or tissue type. Autogenic Living Scaffolds seeded with the appropriate cell types may thus be used to grow implantable tissue and organs *in vitro,* for later implantation into an *in vivo* site.

Many different types of cells may also be seeded in different parts of the Living Scaffold, or they could be sandwiched on top of each other. For example, a Fibroblast Autogenic Living Scaffold may first be grown in serum-free conditions favorable to the growth of the fibroblasts into tissue or an Autogenic Living Scaffold. This Living Scaffold is then seeded with astrocytes, and the serum-free growth conditions (including the media, pH, osmolarity, temperature, oxygen tension, and anything else required) are adjusted to be favorable to the growth of the astrocytes. If needed, other components are added to keep the Living Scaffold alive and healthy. Also, additional layers, such as skeletal muscle myocytes that might form into skeletal muscle tissue that is innervated by the already seeded nerves, may continue to be added, as desired.

In another embodiment, the Autogenic Living Scaffold may also be grown into specific shapes by molds, and may also be reshaped to some degree. For example, a sheet of the above example of a Fibroblast Autogenic Living Scaffold, seeded first with astrocytes and then nerve cells, may be rolled into a cylinder. This cylinder may then be implanted into a spinal cord *in vivo.* The Autogenic Living Scaffold also provides mechanical support to the seeded cells. For example, in a particular embodiment, a Fibroblast Autogenic Living Scaffold seeded with neurons may be mechanically stressed and compressed, without major damage to the neurons, even though such a degree of mechanical stress and compression kills most neurons when grown in the absence of an ALS. The Autogenic Living Scaffold of other embodiments may also be introduced to mechanical stress or tension which may change the properties of the Living Scaffold and any cells or tissue that are growing on it.

In one particular embodiment, the fibers of a Fibroblast Autogenic Living Scaffold may also be made to grow in parallel, which helps seeded nerve cells to also grown in parallel along these fibers, especially when Schwann cells are previously seeded onto the scaffolds and first start growing in parallel along these fibers. This may be even more useful when implanted in the spinal cord, since the implanted nerve cells may then be aligned in the same direction as the native nerve cells in the spinal cord. In still another embodiment, a sheet of Autogenic Living Scaffold with the seeded neurons may also be rolled into a cylinder prior to implantation to produce a structure with layers of neurons aligned in the same general direction as the native neurons in the spinal cord. Similar things may be done for implantation into other tissues and organs.

In other particular embodiments, cell to cell, tissue to tissue, and tissue to cell interactions may also be studied *in vitro* and *in vivo* with Autogenic Living Scaffolds, including by sandwiching different cells. In yet another embodiment, Autogenic Living Scaffolds may be used *as in vitro* biological models for studying the growth and development of cells, tissues, organs, systems, diseases, and different responses in organisms. For example, the wound response (in which fibroblasts play an active role) on different types of cells and tissues may be studied *in vitro* by using this technology.

Fibroblasts (especially foreskin fibroblasts) secrete numerous growth factors including nerve growth factor (NGF), brain-derived neurotrophic factor (BDNF), and neurotrophin-3 (NT-3), as well as fibroblast growth factor (FGF), and platelet-derived growth factor (PDGF), all of which promote neuron regeneration and survival. The embodiments of ALSs described herein more closely mimic the extracellular environment that nerve cells are normally exposed to *in vivo* than any other currently available scaffolds, and even allow primary nerve cells to form active 3-D neural networks *in vitro* that can serve as *in vitro* 3-D models for potential therapeutic agents for neuronal regeneration, as may also be used to functionally replace injured spinal cord neurons *in vivo.*

In the case of particular embodiments of fALS of the present specification the high density of fibroblasts along with the insoluble ECM proteins collagen and fibronectin of the ALS work in conjunction to promote axon elongation and functional recovery when implanted into chronically injured spinal cord. In addition, the development of a functional neural network *in vitro* allows the nerve graft to have more utility when implanted, and is important for studying the effect of different pharmacological agents and methods on the *in vitro* 3-D neural network model disclosed herein.

In other embodiments the effects of different nutritional supplements and growth factors on the development of the functional neural networks in the ALS may also be studied. Thus, in embodiments of the present invention, once the neurons are seeded onto the ALS, the growth media is changed from one that supports ALS growth to one that promotes neuronal growth and differentiation, while at the same time retards the growth of fibroblasts. This prevents the fibroblasts from over-running the neurons and effectively preventing neuronal development. In other embodiments, the fibroblasts may be genetically engineered to secrete more growth factors such as NGF, BDNF, FGF-2, and bFGF to enhance neuron survival and development even further.

In still other embodiments, the ALS nerve graft has the flexibility of taking on almost any non-rigid shape and may be rolled up into a ball or cylinder. Several thin nerve grafts may also be layered on top of each other to form different parallel layers of neural networks, which in turn may again be rolled up into a cylinder or formed into some other shape.

Another embodiment provides an autogenic living scaffold that has been seeded with cells, tissue or combinations thereof, including any of stem cells, progenitor cells, precursor cells; cells or tissue of a connective, epithelial, muscle, nerve and/or glandular origin; and cells of vascular and/or non-vascular organ origin such as neuroblastomas, myoblasts, astrocytes, cardiomyocytes, skeletal muscle myoblasts, hepatocytes, chondrocytes, osteoblasts, fibroblasts, keratinocytes, Schwann cells, nerve cells, glial cells, epithelial cells, endothelial cells, smooth muscle cells, skeletal muscle cells, cardiac muscle cells, stromal cells, mesangial cells, mesenchymal cells, hematopoietic cells, dendritic cells, immune system cells, neural tissue, hepatic tissue, aortic tissue, venous tissue, capillary tissue, cartilage, bone, muscle, glands, and hair follicles.

Another embodiment provides a Living Tissue Matrix (LTM) that closely resembles *in vivo* generative/regenerative connective tissue since the cells produce the entire 3D matrix by themselves. In fact, LTMs are similar in composition to the type of fibroblast-populated connective tissue that first fills the wound bed in embryonic wound healing (and other non-scar forming tissue wound healing) that regenerates without scarring as opposed to wounds in neonatal or adult mammals that heal with scarring. Furthermore, this method produces a 3-D construct (the LTM) that is significantly thicker and stronger than those obtained using biopolymer gels, such as collagen or fibrin gels. The entire Living Tissue Matrix (cells and ECM) can also be made completely autologous, thus preventing host rejection and making it completely immunocompatible.

The ECM produced in the LTM system provides an optimal environment for de-differentiated or transdifferentiated autologous adult cells within the LTMs to create a regenerative environment and a virtual blastema. In other words, if the millions of fibroblasts within the LTM (the cells that produced the LTM in the first place) are de-differentiated or transdifferentiated, the LTM can effectively become a structurally sound implantable blastema-like structure for multi-tissue type regeneration.

The specification describes a chemically defined media formulation (called "fALS Media" or "Matrix Media") for growing an ALS or LTM that contains a 3:1 ratio of DMEM (high glucose - 4.5g/L - with L-glutamine and sodium pyruvate) and Ham's F12 medium (or 2:1 ratio of IMEM to Ham's F12 medium), supplemented with $4.2 \times 10^{-10}$M Epidermal Growth Factor (in human serum albumin); $2.8 \times 10^{-10}$M Basic Fibroblast Growth Factor; $8.6 \times 10^{-5}$M insulin; $1.0 \times 10^{-7}$M dexamethasone; $3.2 \times 10^{-4}$M L-ascorbic acid phosphate magnesium salt *n*-hydrate; $2 \times 10^{-10}$M L-3,3',5-triiodothyronine; $10^{-4}$M ethanolamine or other lipid precursor; $3.9 \times 10^{-8}$M selenious acid; $4 \times 10^{-3}$M Glutamax™; $3.3 \times 10^{-6}$M glutathione (reduced); and 1% penicillin/streptomycin/amphotericin B. In addition, other variations of the above-listed medium may contain additional components, such as any one or more of: Platelet Derived Growth factor (PDGF); 100:68 ratio of glycine: L-proline; L-cysteine; and Trolox. Concentrations may vary as required, as long as the total osmolarity in the medium is kept at acceptable levels for growth of the ALS.

Other embodiments provide methods for growing tissue and/or organs using an ALS or LTM, and methods for using an ALS or LTM to model a human biological cellular system or tissue system of a combination thereof. In addition, methods are provided for using an ALS or LTM to assess the effect of one or more agents on a biological system being modeled, wherein one or more agents includes pharmaceutical agents, enzymes, hormones, small molecules, peptides, polypeptides, natural products, natural products extracts, inorganic salts, other cells, growth factors, clotting factors, toxins, poisons, nucleic acids, mechanical stress inducers, electrical current generators, electromagnetic field and pulse generators, and sonic wave inducers.

The specification also describes using an ALS or LTM to treat tissue or organ damage or tissue or organ degeneration in a subject suffering from Crohn's disease; cancer, including lung, colon, stomach, liver, kidney, pancreas, bone, brain; muscular dystrophy; ocular degeneration, diabetes, cardiac ischemia; heart valve damage or heart valve congenital defect by producing an ALS or LTM, and any one of a) regenerating a new organ or tissue of the tissue *in vitro* using the scaffold and cells or tissue of the type or class of the damaged or degenerated tissue or organ and then implanting such scaffold with regenerated tissue or tissue of the organ in the subject at the site of tissue or organ damage or degeneration ; or b) implanting the scaffold and cells or tissue of the type or class of the damaged or degenerated tissue or organ in the subject at the site of tissue or organ damage or degeneration and then regenerating a new organ or tissue of the organ *in vivo* using the scaffold having cells or tissue of the type or class of the damaged or degenerated tissue or organ; or c) regenerating a new organ or tissue of the organ as in a) or b) using the scaffold alone.

Other embodiments provide methods for using an ALS to generate viable vertebrate neuronal tissue *in vitro* comprising seeding an ALS with vertebrate primary neural cells or neuronal tissue and maintaining the seed scaffold in culture under conditions where viable vertebrate neuronal tissue is generated. Such viable vertebrate neuronal tissue is then suitable for treating paralysis in a subject by contacting at least one site of spinal cord in the subject with an effect amount of the ALS with viable neuronal tissue so as to treat the paralysis.

The specification also describes treating a neurodegenerative disease in a subject, wherein the neurodegenerative disease is any one of Parkinson's disease, Huntington's disease, Alzheimer's disease, schizophrenia, dementia, multiple sclerosis, cerebral palsy, muscular dystrophy, Tay Sach's disease, Mad Cow disease, or Creutzfield-Jacob's disease, by contacting at least one site in the subject with an effective amount of an ALS or LTM with viable neuronal tissue so as to treat the neurodegenerative disease.

## Brief Description of the Drawings

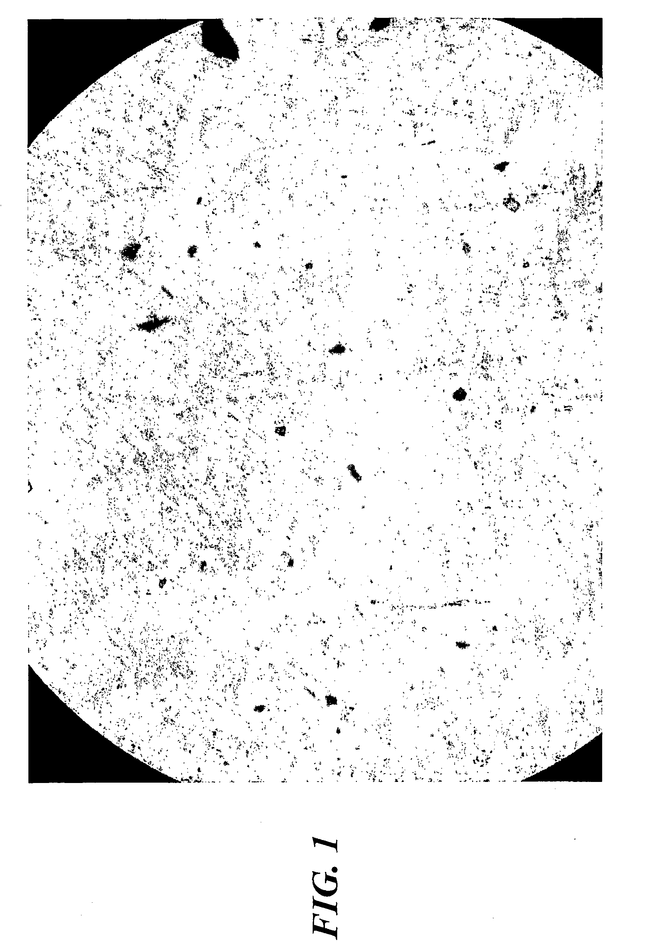

**Fig.1** shows methylene blue staining of neurons that have differentiated from neural progenitor cells grown on a fibroblast ALS.

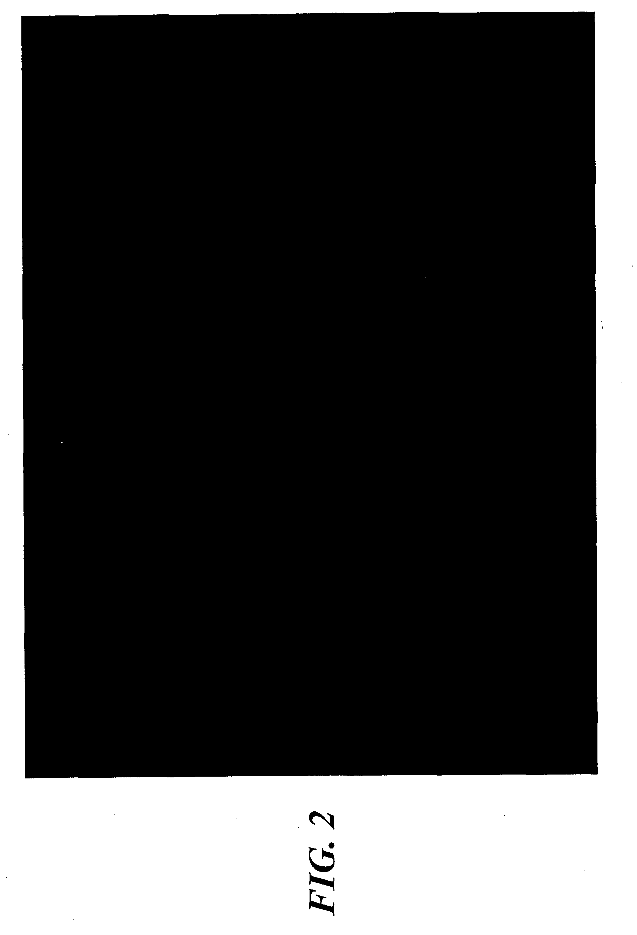

**Fig. 2** shows a fluorescent live/dead assay performed on neural progenitor cells grown on a fibroblast ALS, where the fibroblasts had been killed prior to seeding with human neural progenitor cells. Live neurons (nerve and glial cells) fluoresce as green; the nuclei of dead cells (mostly fibroblasts) fluoresce as red.

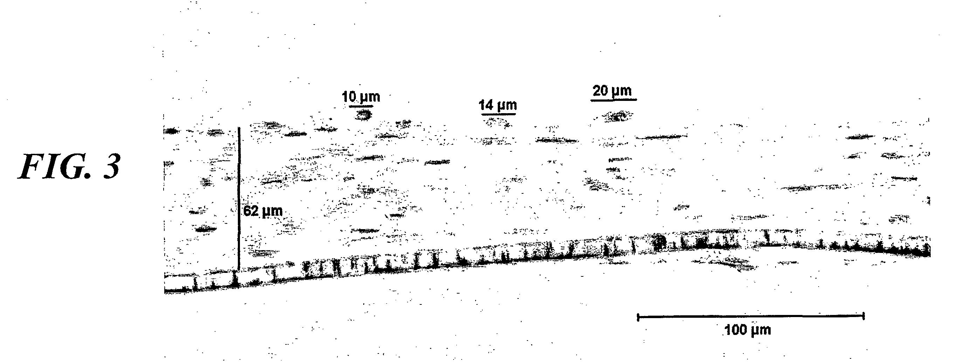

**Figs. 3** shows a cross-section of a 17-day fibroblast ALS system about 50-60 $\mu$m thick containing neuronal cells/tissue that are expressing anti-Hu MAb 16A11, an early marker of vertebrate neurogenesis that is expressed shortly after neuronal terminal mitosis (Marusich, M.F. et al (1994) J. Neurobiol. 25 : 143-155). The marker is observed in nerve cell bodies as reddish brown spots; cell nuclei are blue. The ALS was allowed to grow for 4 weeks prior to seeding with human neuroprogenitor cells.

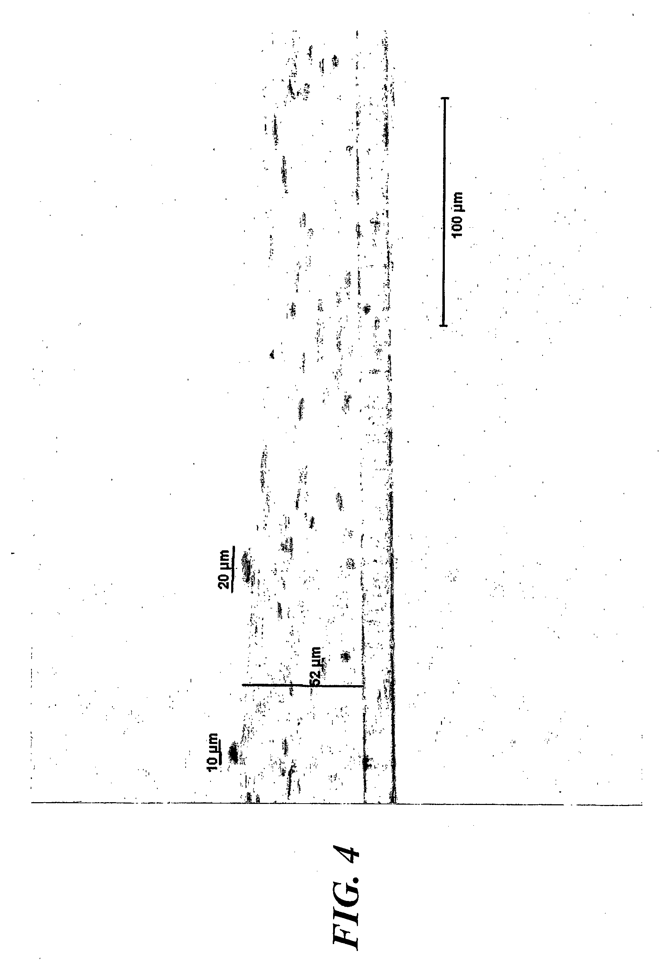

**Fig. 4** shows another cross-sections of a 17-day fibroblast ALS system about 50-60 $\mu$m thick containing neuronal cells/tissue that are expressing anti-Hu MAb 16A11, an early marker of vertebrate neurogenesis that is expressed shortly after neuronal terminal mitosis (Marusich, M.F. et al (1994) J. Neurobiol. 25 : 143-155). The marker is observed in nerve cell bodies as reddish brown spots; cell nuclei are blue. The ALS was allowed to grow for 4 weeks prior to seeding with human neuroprogenitor cells.

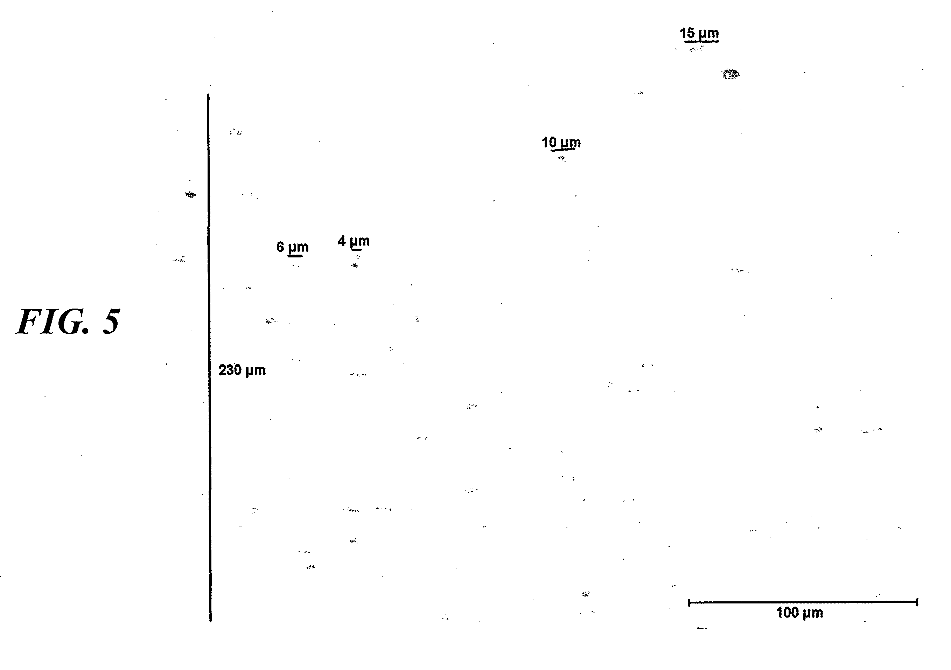

**Fig. 5** shows cross-sections of a 31-day fibroblast ALS system about 216-230 $\mu$m thick containing neuronal cells/tissue that are expressing anti-Hu MAb 16A11. The ALS was allowed to grow for 3 weeks prior to seeding with human neuroprogenitor cells. As can be seen, varying-sized regions (different cross-sections of nerve cell bodies) expressing the anti-Hu MAb 16A11 marker are indicated, ranging in size to over 20 $\mu$m.

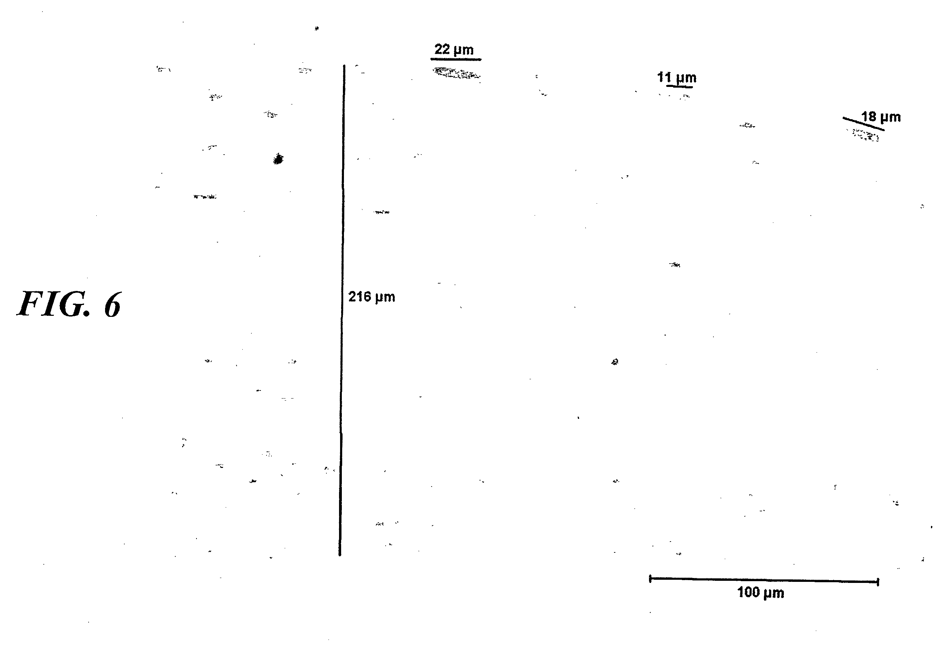

**Fig. 6** shows another cross-sections of a 31-day fibroblast ALS system about 216-230 $\mu$m thick containing neuronal cells/tissue that are expressing anti-Hu MAb 16A11. The ALS was allowed to grow for 3 weeks prior to seeding with human neuroprogenitor cells. As can be seen, varying-sized regions (different cross-sections of nerve cell bodies) expressing the anti-Hu MAb 16A11 marker are indicated, ranging in size to over 20 $\mu$m.

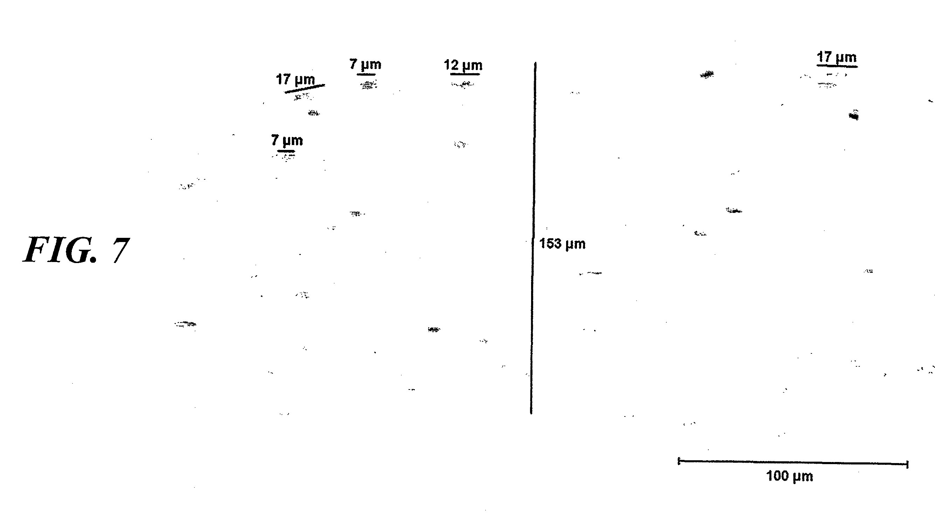

**Fig. 7** shows cross-sections of a 24-day fibroblast ALS system about 153 $\mu$m thick containing neuronal cells/tissue that are expressing anti-Hu MAb 16A11. The ALS was allowed to grow for 2 weeks prior to seeding with human neuroprogenitor cells. As can be seen, varying-sized regions (different cross-sections of nerve cell bodies) expressing the anti-Hu MAb 16A11 marker are indicated, ranging in size to almost 20 $\mu$m.

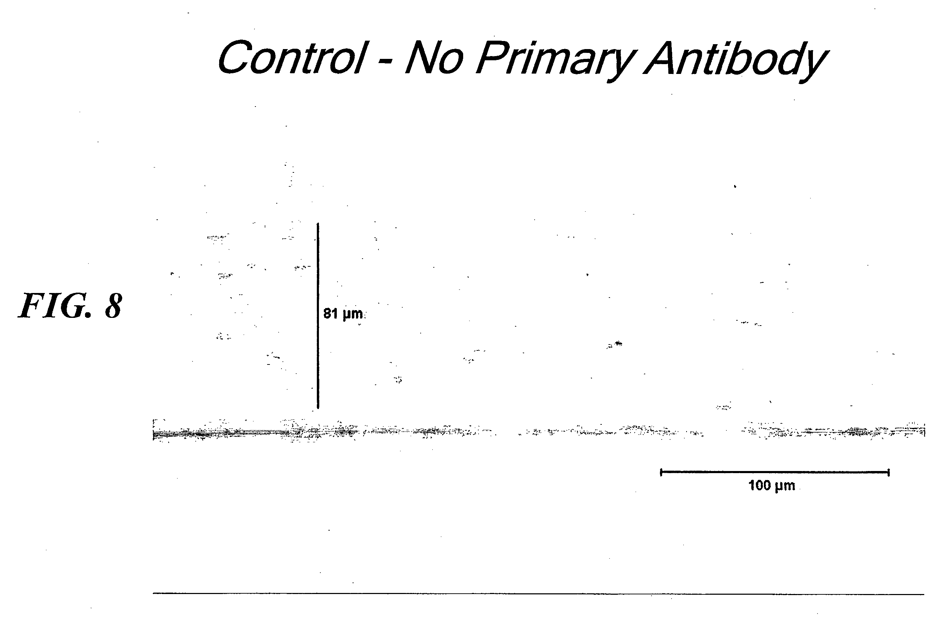

**Fig. 8** shows a section view of a negative control system for the neurogenesis marker experiments of Figures 3 through 7, where no primary Hu MAb 16A11 antibody was present in the system. Thus, only the nuclei (in blue) of the cells are visible, and no expressed neurogenesis markers are visible.

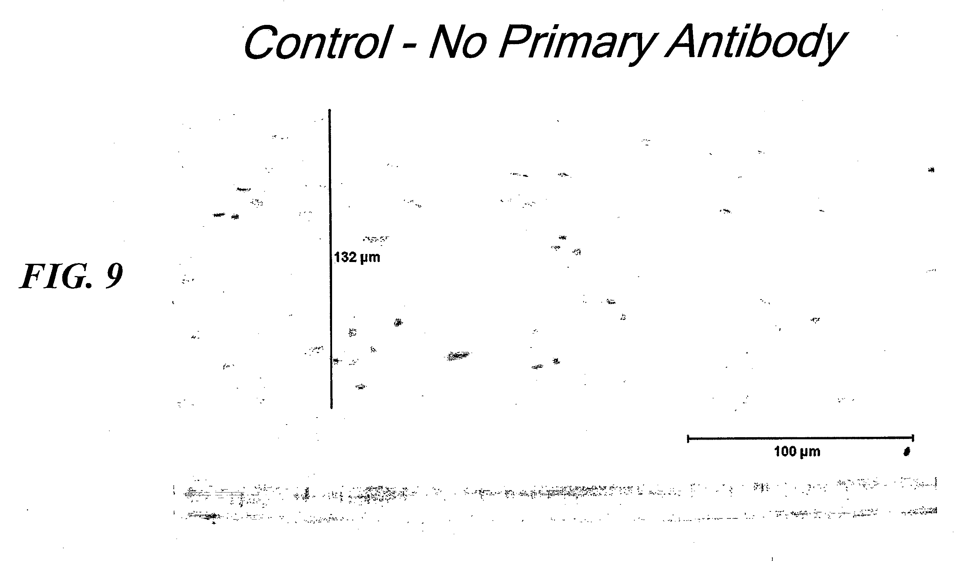

**Fig. 9** shows a different section view of a negative control system for the neurogenesis marker experiments of Figures 3 through 7, where no primary Hu MAb 16A11 antibody was present in the system. Thus, only the nuclei (in blue) of the cells are visible, and no expressed neurogenesis markers are visible.

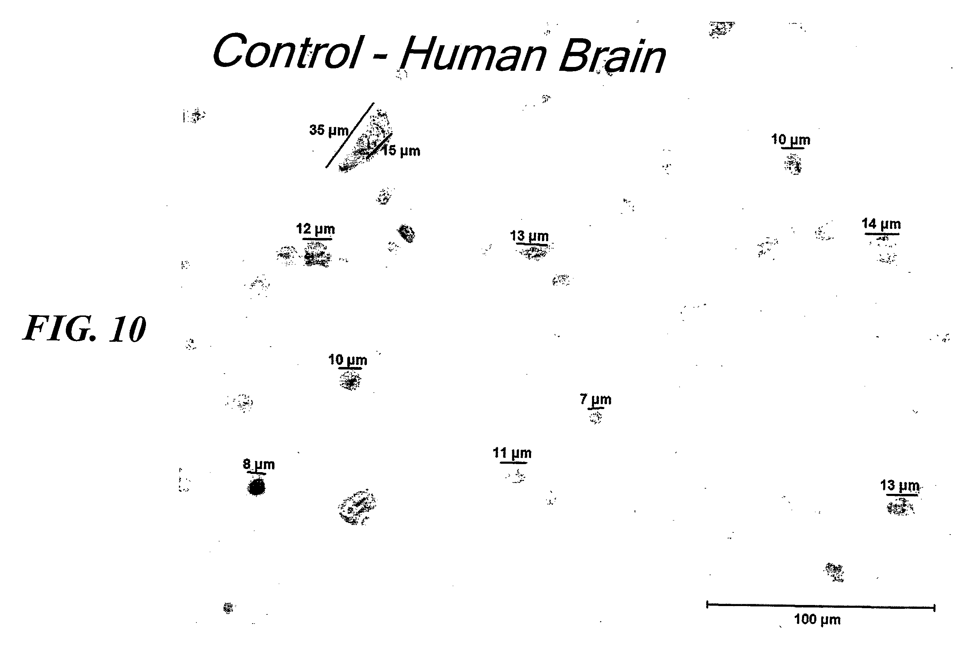

**Fig.10** shows a cross-sectional view of a positive control system for the neurogenesis marker experiments of Figures 3 through 7, using human brain sections. Positive indication that the marker anti-Hu MAb 16A11 attaches to nerve cell bodies is indicated by the large and numerous brown dye spots visible throughout the images. Most of the cells that did not stain for anti-Hu MAb 16A11 are glial cells.

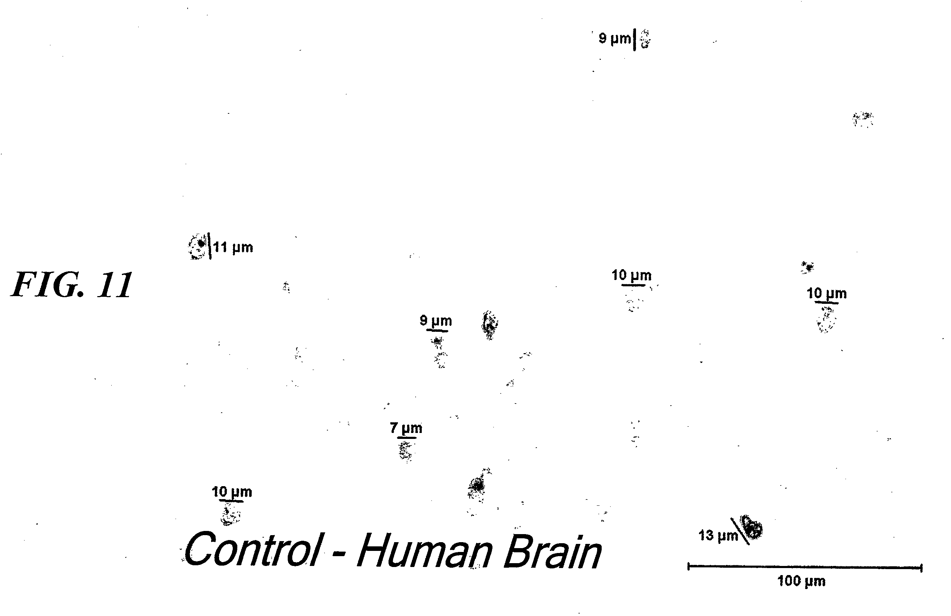

**Fig. 11** shows a different cross-sectional view of a positive control system for the neurogenesis marker experiments of Figures 3 through 7, using human brain sections. Positive indication that the marker anti-Hu MAb 16A11 attaches to nerve cell bodies is indicated by the large and numerous brown dye spots visible throughout the images. Most of the cells that did not stain for anti-Hu MAb 16A11 are glial cells.

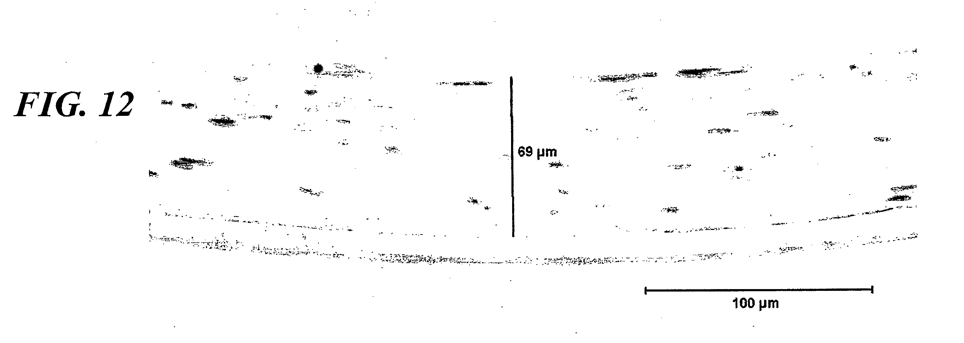

**Fig. 12** shows trichrome staining of a cross-section of a 24-day fibroblast ALS system containing muscle cells/tissue about 70 $\mu$m thick. The collagen fibers of the ALS are blue; the nuclei of cells are (bluish) black; and muscle cells (differentiated from human skeletal muscle myoblasts seeded onto the ALS 2 weeks earlier) within the ALS are observed as reddish spots within the ALS sections. The ALS was allowed to grow for 1 week prior to seeding with human skeletal muscle myoblasts.

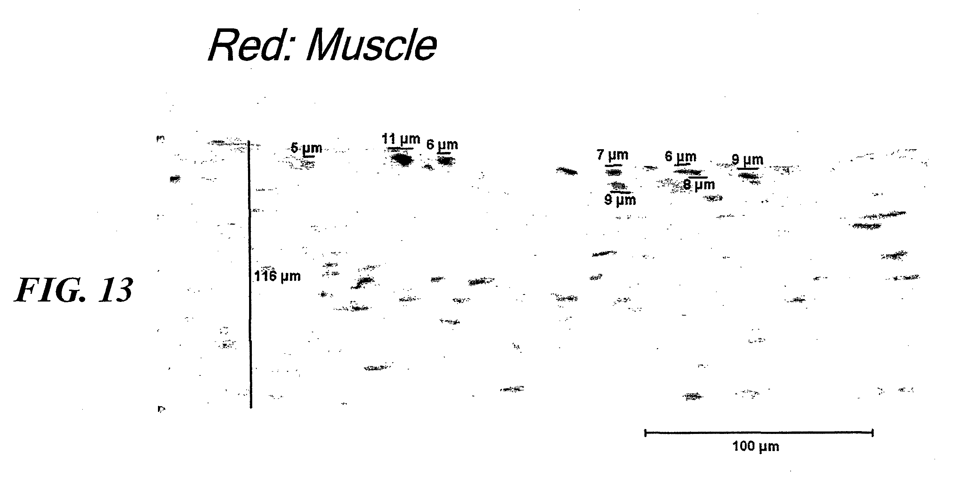

**Fig. 13** shows trichrome staining of a different cross-section of a 24-day fibroblast ALS system containing muscle cells/tissue about 115 $\mu$m thick. The collagen fibers of the ALS are blue; the nuclei of cells are (bluish) black; and muscle cells (differentiated from human skeletal muscle myoblasts seeded onto the ALS 2 weeks earlier) within the ALS are observed as reddish spots within the ALS sections. The ALS was allowed to grow for 1 week prior to seeding with human skeletal muscle myoblasts.

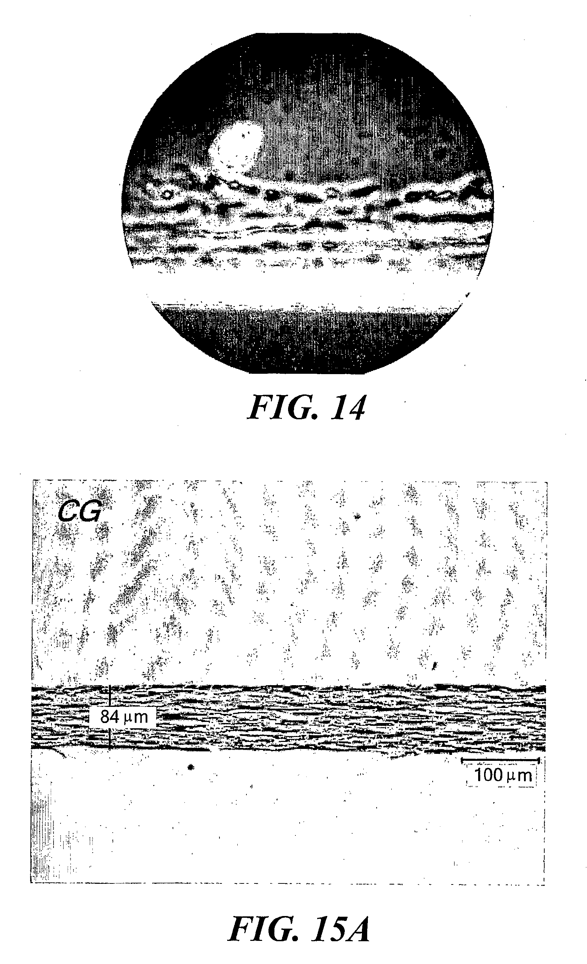

**Fig. 14** shows early nerve differentiation on Living Tissue Matrix. Human neural cell bodies stained with Hu16A11 (brown), a marker of early neurogenesis.

**Fig. 15A** shows Hematoxylin and Eosin (H&E) stained sections of fibroblast-populated collagen gel (CG), 84 $\mu$m thick, as measured by digital image analysis.

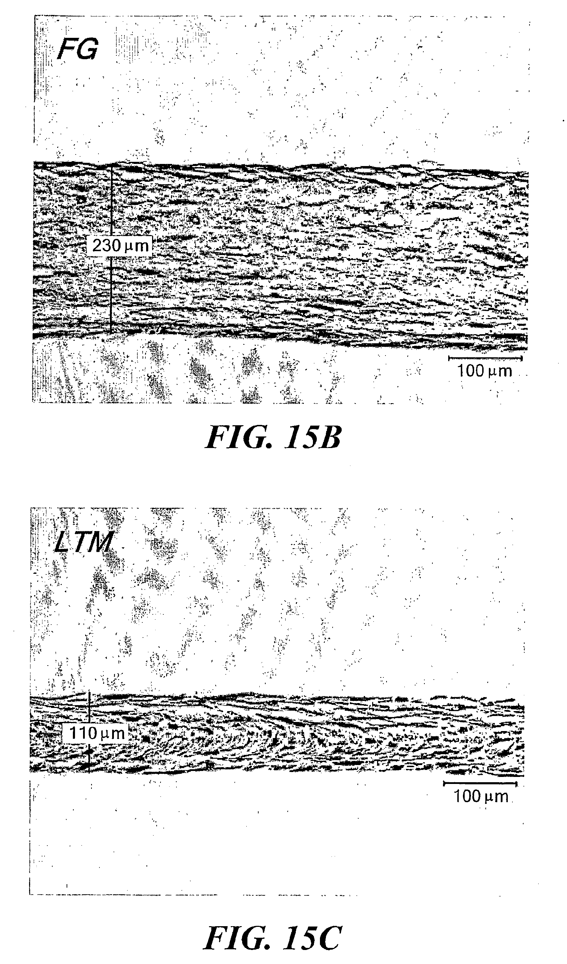

**Fig. 15B** shows fibroblast-populated fibrin gel (FG), 230 $\mu$m thick, as measured by digital image analysis.

**Fig. 15C** shows Living Tissue Matrix (LTM) fed with the same serum-supplemented media that the CG and FG above were fed with, 110 $\mu$m thick, as measured by digital image analysis.

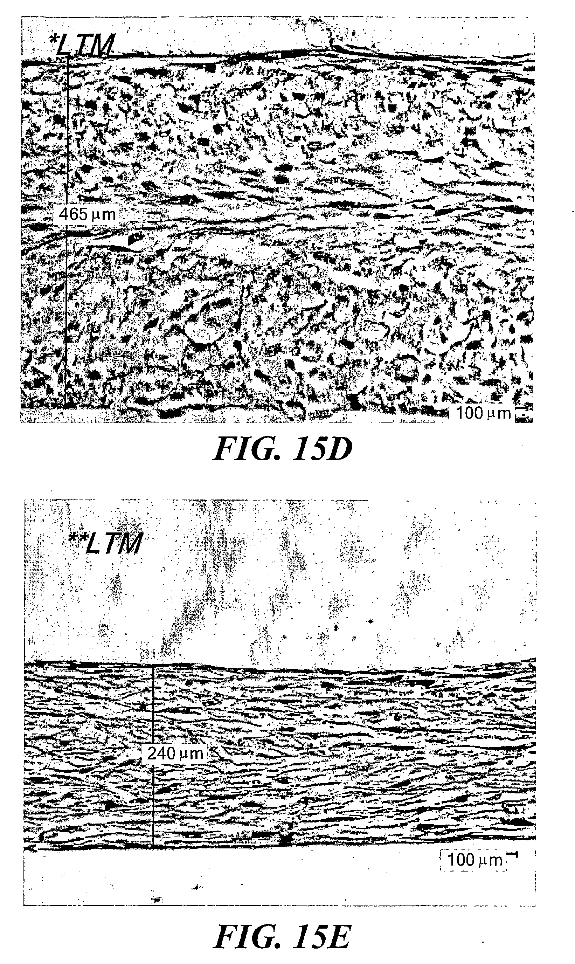

**Fig. 15D** shows Living Tissue Matrix (*LTM) fed with Matrix Media, 465 $\mu$m thick, as measured by digital image analysis.

**Fig. 15E** shows Living Tissue Matrix (**LTM) fed with a modified Matrix Media, 240 $\mu$m thick, as measured by digital image analysis. All micrographs for Figures A-E were taken at 200x. Scale bars =100 $\mu$m.

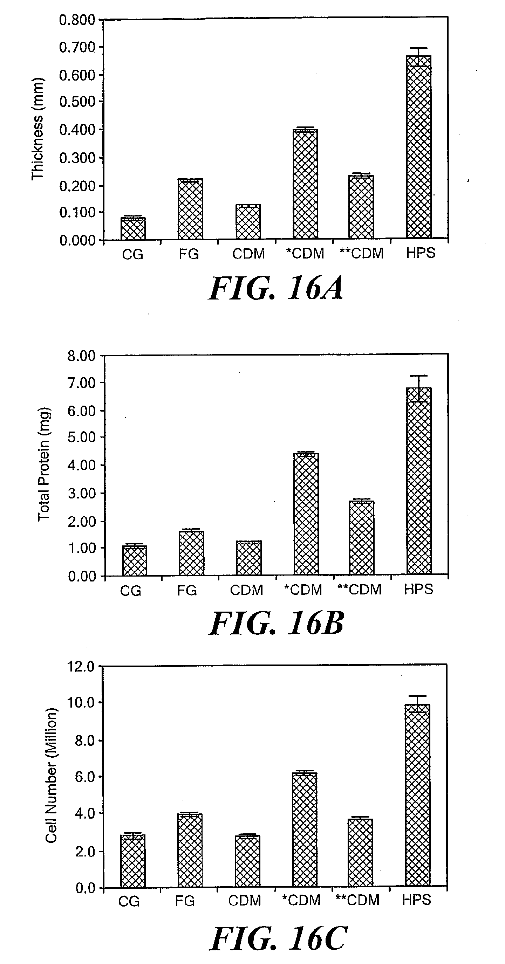

**Fig. 16A** is a graphical representation of the results in Table 1 showing the thickness ($\mu$m) of the fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

**Fig.16B** is a graphical representation showing the tensile strength (N/m) of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

**Fig. 16C** is a graphical representation of the results in Table 1 showing the total collagen (mg) of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

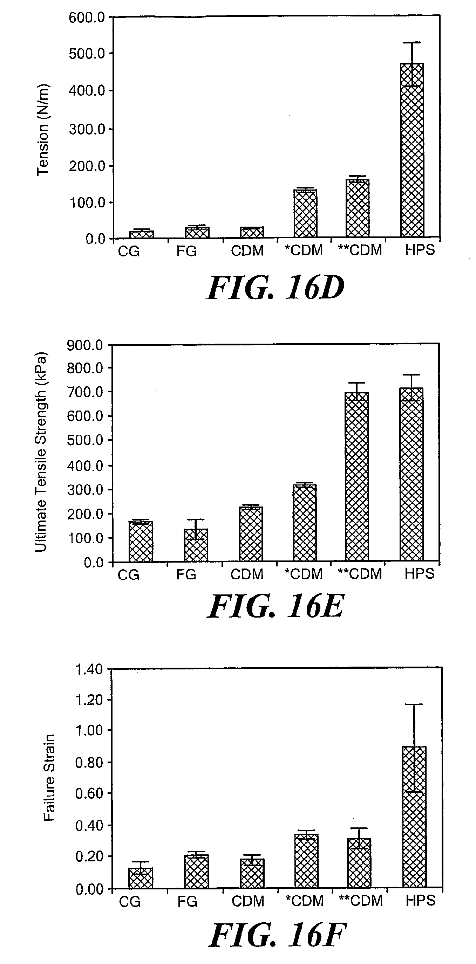

**Fig. 16D** is a graphical representation of the results in Table 1 showing the total proteoglycans and glycosaminogly-cans ($\mu$g) of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

**Fig. 16E** is a graphical representation of the results in Table 1 showing the total protein (mg) of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

**Fig. 16F** is a graphical representation of the results in Table 1 showing the ultimate tensile strength (kPa) of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

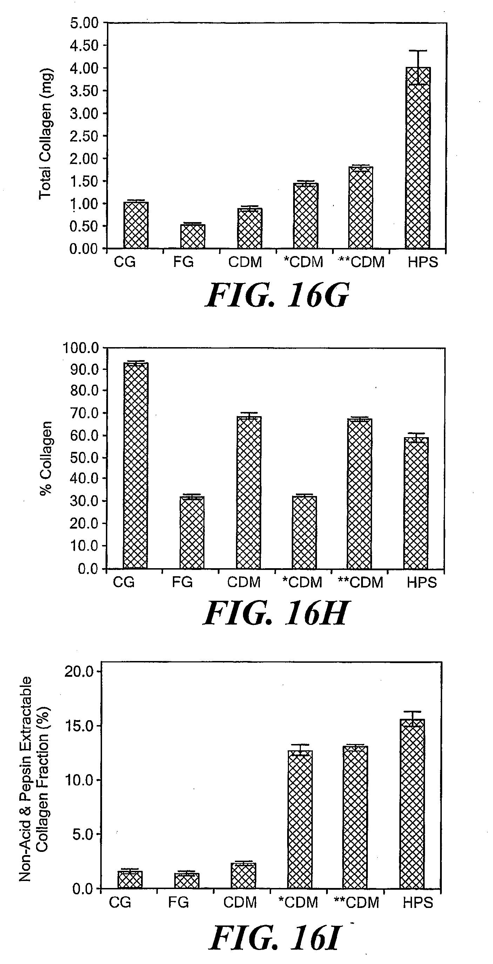

**Fig. 16G** is a graphical representation showing the % collagen of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

**Fig. 16H** is a graphical representation showing the % proteoglycans and glycosaminoglycans of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

**Fig. 16I** is a graphical representation of the results in Table 1 showing the cell number (millions) of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

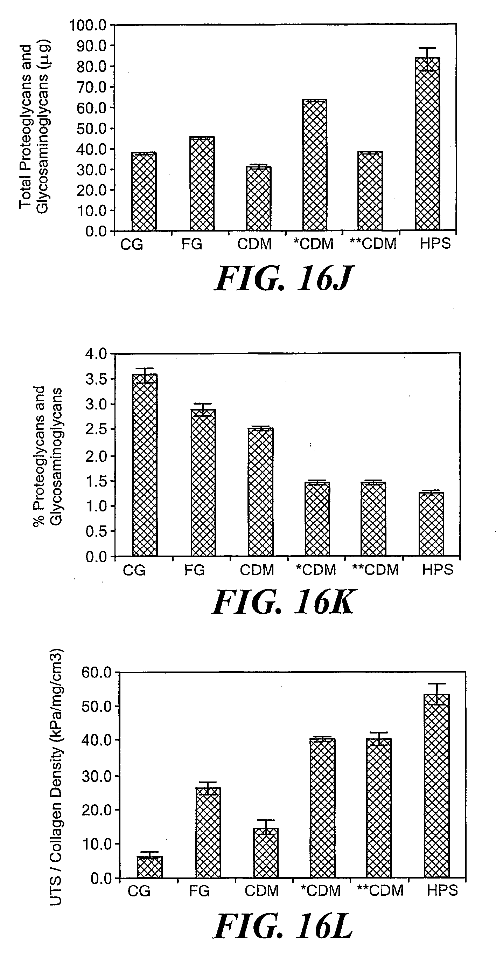

**Fig. 16J** is a graphical representation of the results in Table 1 showing the failure strain of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

**Fig. 16K** is a graphical representation of the results in Table 1 showing the non-acid and pepsin extractable collagen fraction (%) of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

**Fig. 16L** is a graphical representation of the results in Table 1 showing the UTS/collagen density (kPa/mg/cm$^3$) of fibroblast-populated collagen gel (CG) and fibrin gel (FG), and cell-derived matrix (CDM) fed with serum-supplemented medium; and cell-derived matrices (*CDM and **CDM) fed with Matrix Media, compared to human penile skin (HPS).

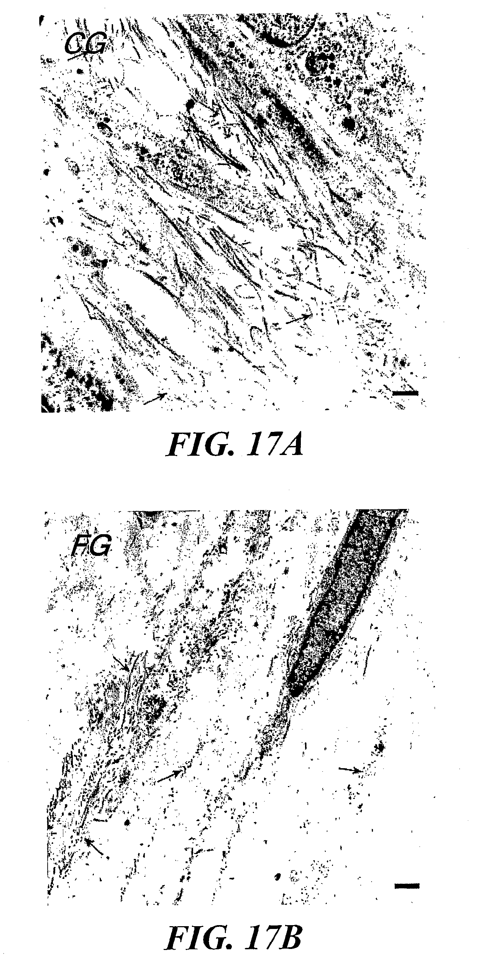

**Fig. 17A** shows a TEM 12,000x; scale bar = 1 $\mu$m) of the distribution of collagen fibrils (arrows) in the collagen gel (CG), wherein most (from the collagen gel) are about 56 $\pm$ 5 nm in diameter with a small number (presumably secreted by fibroblasts in the gel) about 46 $\pm$ 5 nm in diameter. The collagen density was 81 $\pm$ 4 fibrils/$\mu$m$^2$.

**Fig. 17B** shows a TEM 12,000x; scale bar = 1 $\mu$m) of the distribution of collagen fibrils (arrows) in the fibrin gel (FG) wherein the collagen fibrils have a diameter of about 48 $\pm$ 5 nm in diameter. The collagen density was 28 $\pm$ 4 fibrils/$\mu$m$^2$, (p<0.01).

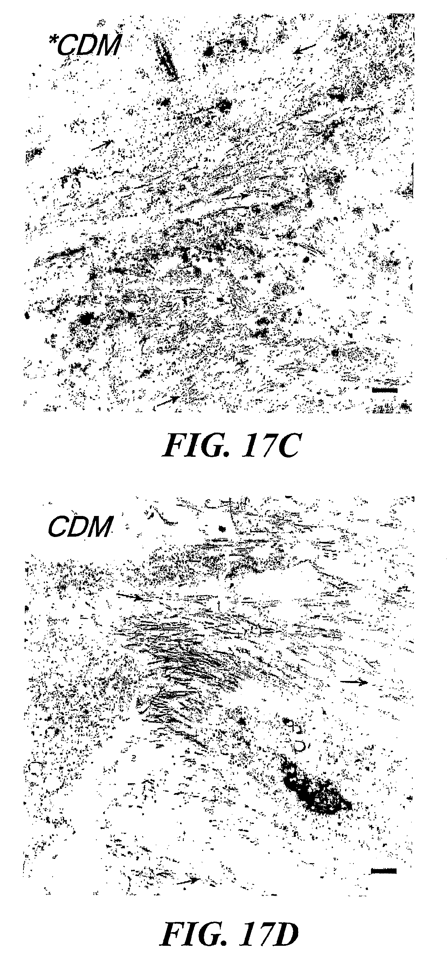

**Fig. 17C** shows a TEM (12,000x; scale bar = 1 $\mu$m) of the distribution of collagen fibrils (arrows) in the cell-derived matrix (CDM) wherein the collagen fibrils have a diameter of about 52 $\pm$ 10 nm in diameter. The collagen density was 79 $\pm$ 2 fibrils/$\mu$m$^2$.

**Fig. 17D** shows a TEM 12,000x; scale bar = 1 $\mu$m) of the distribution of collagen fibrils (arrows) in the cell-derived matrix (*CDM) fed with Matrix Media wherein the collagen fibrils have a diameter of about 47 $\pm$ 5 nm in diameter. The collagen density was 80 $\pm$ 2 fibrils/$\mu$m$^2$.

## Detailed Description of Specific Embodiments

Definitions. As used in this description and the accompanying claims, the following terms shall have the meanings indicated, unless the context otherwise requires:

"Autogenic Living Scaffold", or "ALS" as used herein means a 3-dimensional structure comprising cells (or entities) and the ECM (or matrix) that has been completely produced and arranged by these cells (or entities) that supports the growth of cells, tissue, organs, or combinations thereof. "Autogenic" means it is a self-produced scaffold, providing mechanical and nutritional and developmental support for the cells, tissues, organs, or combinations thereof. "Living" means that the scaffold is smart - it adjusts to its environment. "Living" also means it is biologically active and all components except seeded cells and tissue are naturally formed, produced, and synthesized by the scaffold system itself - i.e., the scaffold is autogenic. "Scaffold" means that it provides a structural framework for cells that guide their direction of growth, enables them to be correctly spaced, prevents overcrowding and enables cells to communicate between each other, transmit subtle biological signals, receive signals from their environment, and form bonds and contacts that are required for proper functioning of all cells within a unit such as a tissue.

"Resembles" as used herein means there is physical, functional, compositional, structural, phenotypic or other similarities between the materials or systems being compared, such that the objects are substantially equivalent. "Substantially equivalent" means that visible, microscopic, physical, functional, and other observations and assays do not easily or significantly distinguish the materials or systems. An easy or significant distinction would, for example, be a functional difference, a physical difference, a compositional difference, a structural difference immediately apparent, or easily detectable with standard assays and observational techniques such as staining, microscopy, antibodies, etc.

"Extracellular Matrix" (ECM) or "Cell Derived Matrix" (CDM) or Cell-produced Matrix as used interchangeably herein means a cell-derived secreted structural substance produced by and/or secreted from cells into the extracellular space. The ECM/CDM provides a growth template for any cell type to grow, differentiate, and produce tissue. Any cell type, as used herein, includes stem cells, progenitor cells, differentiated cells, and any other type of cell or entity. The term also is intended to include the matrix material referred to as Extracellular Growth Matrix (ECGM) that is produced by Radicari entities, and Radicari pre-cells and cells, as described in US application Serial Number 10/930,673 filed August 30, 2004 which claims priority from US provisional application Serial Number 60/499,142 filed August 29, 2003. The ECM allows cell attachment and cell migration, and promotes cell differentiation. The ECM also aids the formation of new tissue of a desired or existing cell type As used herein means, "Cell-Produced Matrix, also called Cell-Derived Matrix (CDM)" also means a 3-dimensional ECM (or matrix) structure that has been completely produced and arranged by cells (or entities) in vitro.

"Construct" as used herein means a physical structure with mechanical properties such as a matrix of scaffold Construct encompasses both autogenic living scaffolds and living tissue matrices, ex-vivo cell-produced tissue and cell-derived matrix.

"Cell-derived" as used herein means that the source for the material, body, or component is a cell or a collection of cells.

"Ex-vivo Cell-produced Tissue (ECT)" as used herein means, a functional tissue comprising one or more types of cells (or entities) and the ECM (or matrix) that has been completely produced and arranged by some of these cells (or entities). For example, an ex-vivo cell-produced neural graft consisting of an ALS that has been seeded with neuroprogenitor cells that differentiated in the ALS and formed neural tissue.

"Living Tissue Matrix (LTM)" as used herein means, a 3-dimensional tissue (or matrix) that is capable of being transformed into a more complex tissue (or matrix) or a completely different type of tissue (or matrix) that consists of cells (or entities) and the ECM (or matrix) that has been completely produced and arranged by these cells (or entities).

"Living Tissue Equivalent (LTE)" as used herein means, a construct containing living cells that intends to mimic a certain type of native tissue. This construct can be produced by any means in vitro, including by the use of artificial scaffolds.

"Superconfluent conditions" or "Superconfluency" means, within the context of the present application, that cells are grown and maintained in high-density growth conditions such that cells are packed almost directly next to and top of each other.

"Hyperconfluent conditions" as used herein means conditions for *in vitro* cell culture/growth such that cells and their associated ECM take up all the space at the bottom of the culture dish and have started to grow in the third dimension (on top of each other).

"Base media" as used herein means any cell culture medium that supports *in vitro* cultures of eukaryotic cells, including Dulbecco's Modification of Eagle's Medium (DMEM); Ham's F-12 (F12); Ham's F-10 (F10); Iscove's Modification of DMEM (IDMEM); MEDIUM 199; Alpha MEM; Basal Medium Eagle with Earle's BSS; Cyroprotective Medium (Freezing Medium); DMEM:F12 1:1; with L-Glutamine; Glasgow's MEM with L-glutamine (GMEM); IMDM with HEPES, with or without L-Glutamine; L-15 (Leibovitz), without L-Glutamine; McCoy's 5A Modified Medium; Medium 199; MEM Eagle; MEM Eagle-Earle's BSS, with or without L-Glutamine; MEM Eagle-Hanks BSS; NCTC-109; Richter's CM Medium; RPMI 1640 with HEPES; RPMI 1640; or a combination of any of these.

"Glucocorticoid" as used herein, means dexamethasone, hydrocortisone, corticosterone, cortisol, cortisone, prednisone, prednilisone, methylprednisone, budesonide, beclometasone or any other compound classified as, or commonly referred to as, a glucocorticoid, or which interacts with the glucocorticoid receptor.

"Growing a large number of fibroblasts" as used herein means growing a plurality of cultures of fibroblasts to obtain at least about 1,000 cells/mm$^3$ to greater than 200,000 cells/mm$^3$, or growing a single culture to obtain at least about 1,000 cells/mm$^3$ to greater than 200,000 cells/mm$^3$.

"Growth and development" as used herein, means ability to reproduce and be viable, and includes proliferation and differentiation and/or tissue development.

"Fragility" as used herein, means a cell's tendency to be easily broken or damaged, and refers to a cell's physical frailty and weakness, lack of structural hardiness, and inability to remain intact if subjected to physical stress.

"Conditions that promote three-dimensional tissue growth" as used herein, means *in vitro* or *in vivo* conditions that facilitate, aid, further or in any way allow the development of three-dimensional tissue growth. Conditions may include use of specific media, growth factors, minerals, incubation temperature, cell density, aeration, agitation, use of ALS "molds" to shape and contain growth of desired tissue, use of subatmospheric pressure chambers such as Synthecon™ near-zero-gravity incubator systems (such as HARVs and STLVs) for growth of desired tissue, use of microcarrier beads, use of natural or biodegradable scaffolds, implanting a non-fibroblast-seeded autogenic living scaffold within an *in vivo* site such as in an organ or tissue such as connective, epithelial, muscle, and/or nerve tissue.

An "equivalent growth factor" as used herein means any growth factor that can replace a specific growth factor in a fibroblast cell culture without rendering the fibroblast cells non-viable. For epidermal growth factor (EGF), such equivalent growth factors include, but are not limited to, basic fibroblast growth factor (bFGF), fibroblast growth factor 2 (FGF-2), and transforming growth factor-α (TGF-α)

"Concentrations supportive of fibroblast growth and production of extracellular matrix" as used herein, means concentrations of growth media components, whether determined by molarity, weight/volume percent, weight/weight percent, volume/volume percent or any other standard means for measuring concentration, which allow fibroblast cells to grow, reproduce, proliferate, differentiate, or in any other way remain viable and produce extracellular matrix material.

"Regenerate tissue" as used herein means that autogenic living scaffolds with or without seeded cells and/or tissue, form, create, construct or otherwise generate tissue anew where previously there was none, or where previously the tissue was partially or completely non-functional.

"Supports the growth" as used herein means that growth is compatible with, and/or promoted by, the system of interest. For example, the phrase "the scaffold supports the growth of cells" or "supports the growth of tissue" or "supports the growth of organs" means that the ALS is compatible with, and/or promotes, the growth of cells, tissue or organs within or on or throughout the scaffold.

"Genetically engineered" as used herein means that a cell or entity, by human manipulation such as chemical, physical, stress-induced, or other means, has undergone mutation and selection; or that an exogenous nucleic acid has actually been introduced to the cell or entity through any standard means, such as transfection; such that the cell or entity has acquired a new characteristic, phenotype, genotype, and/or gene expression product, including but not limited to a gene marker, a gene product, and/or a mRNA, to endow the original cell or entity, at a genetic level, with a function, characteristic, or genetic element not present in non-genetically engineered, non-selected counterpart cells or entities.

"Smart" as used herein in regards to the autogenic living scaffold means that the living scaffold adapts to its environment, changes relative to its situation and surroundings, in both a reactive and active manner, interacting with and reacting to surrounding factors, cells, entities and structures through physical, biochemical, cell-signaling, enzymatic and/or genetic means. Being smart may also include morphing into a more complex, biochemically-, physically- and/or genetically- evolved material, system, scaffold or matrix; evolving functionally, physically, biochemically and/or genetically; and integrating functionally, phycically, biochemically and/or genetically with surround cells, entities and materials.

"Cell or cell type suitable for production of extracellular growth matrix" or "produced by any suitable cell type" as used herein means any cell, cell type, or cell entity, including Radicari entities and Radicari pre-cells and Radicari cells derived therefrom, that can be manipulated to produce extracellular matrix *in vitro.* "Manipulated to produce extracellular matrix *in vitro"* means that growth conditions, growth media, supplemental factors, and appropriate stimuli exist and may be applied to these cells and entities to stimulate production of extracellular matrix material, in contrast to non-suitable cell types and cell entities wherein conditions, media, factors and stimuli do not exist and cannot be applied to stimulate production of extracellular matrix material.

"Lipid precursor" as used herein means a small molecular precursor in lipid synthesis or biosynthesis that forms the structural backbone for connection of the fatty acid chains. Examples include ethanolamine or equivalents such as *o*-phosphorylethanolamine, and also include any others that are interchangeable with ethanolamine in a serum-free medium for growing the ALS as herein disclosed.

The "Autogenic Living Scaffolds" (ALS) as disclosed herein are made and composed of living cells and the extracellular matrix (ECM) that these living cells produce. The living cells that these Autogenic Living Scaffolds contain may be genetically engineered or otherwise modified. They serve as blueprints, supporting structures, backbones, or scaffolds for the same or other cell lines or types. The ALS may also provide proper or supporting mechanical and chemical environments, signals, or stimuli to other cells, to the cells that produce the ALS, to surrounding tissue at an implantation site, to a wound, or for in vitro generation and regeneration of cells, tissue and organs. They may also provide other cells with nutrients, growth factors, and other necessary or useful components. They may also take in or serve as buffers for certain substances in the environment, and have also some potential at adapting to new environments.

The fibroblast produced matrix of the ALS, or "cell-derived matrix" (CDM), is composed of a number of insoluble extracellular matrix (ECM) molecules including fibronectin, collagen-1 and collagen-3, possibly in ratios resembling those *in vivo.* ALS could thus provide a more "natural" environment, or an environment more closely resembling that of native tissue *in vivo,* for both fibroblasts and other cells seeded onto them *in vitro,* than artificial scaffolds and reconstituted extracellular matrices such as collagen gels provide. The ALS would thus potentially allow more accurate *in vitro* simulation of *in vivo* conditions to aid in better design of medical treatments and *in vitro* model systems.

In one embodiment, ALS is composed of slowly proliferating multilayered fibroblasts surrounded by dense extracellular matrices produced by the fibroblasts, a large proportion of which consists of supermolecularly organized collagen. The fibroblasts in this self-produced mechanically stressed environment assume a synthetic phenotype. The synthetic phenotype of fibroblasts is the phenotype most commonly found in connective tissue *in vivo,* and is an activated cell phenotype characterized by low cell proliferation, high collagen accumulation, fibrillar fibronectin organization, and the formation of actin stress fibers and focal adhesions (Kessler *et al.,* 2001). This allows the ALS to be strong enough to provide structural support for other cell types seeded onto the matrix (and thus serve as a scaffold). The slow proliferation rate of the fibroblasts in the ALS keeps the fibroblasts from overgrowing other cell types. Changing the culture media from one that promotes fibroblast growth to one that promotes the growth and development of subsequently seeded cells (while at the same time possibly retarding the growth of the fibroblasts), also helps to ensure that the fibroblasts do not overgrow the seeded cells and/or tissue.

A chemically defined media allows more control of the ALS growth parameters (and thus its mechanical and chemical properties), and allows the elimination of non-human components in the *in vitro* biological model and scaffold for cell differentiation. Several chemically defined media for fibroblast have been formulated over the years (Bettger *et al.,* 1981; Shipley and Ham, 1983; Parenteau, 1994; Vaccariello *et al.,* 1999). None allows the efficient production of high-strength, high density, fast-growing ECM, while simultaneously keeping the growth of the fibroblast cells themselves sufficiently slow so that the fibroblasts do not over-run other cells seeded into the system. The ALS Medium allows the proper production of an autogenic "living" scaffold (ALS) system for producing cells and tissue of interest *in vitro,* for later implantation *in vivo*, wherein all components of the system are entirely defined, free from non-human components, and the ALS thus-formed is of sufficient strength, adaptability, and thickness to facilitate the growth of cells and tissue of interest in an efficient and economical manner.

Autogenic Living Scaffolds made up of fibroblasts (these may be only a few cells thick, or over 1 mm in thickness) and other similar classes of cells (most of the anchorage-dependent class of cells) such as stromal cells, especially those grown in serum-free conditions and whose growth rate and other characteristics may be controlled, may also serve another important role when implanted *in vivo.* For example, Fibroblast Autogenic Living Scaffolds (fALS) can grow and attach themselves to the site of implantation within a period of hours to days, and thus help to keep the implanted cells stationary with respect to the implantation site. This is very important, for example, in nerve grafts where a fraction of a degree change in the direction of a growing axon could/may prevent the nerves from making efficient connections with the existing nerves adjacent to the site of implantation. Fibroblast ALSs also have the capacity to adjust to their environments, making them "smart". For example, when a damaged portion of a heart is cut out and a fibroblast- or myofibroblast-ALS seeded with cardiomyocytes and/or skeletal muscle myocytes is implanted into this site, the fibroblasts or myofibroblasts grow and attach themselves to the remaining portions of cardiac muscle around the site of the incision in a relatively short period of time and keep the scaffold almost completely stationary relative to the surrounding cardiac muscle. The cardiomyocytes or skeletal muscle myocytes (or other seeded cells) then grow and develop into new cardiac muscle or into comparable cardiac muscle replacements that attach and join to the existing cardiac muscle. Using fibroblast- or myofibroblast-ALSs that are high in Collagen III relative to Collagen I (as is the case with the secreted ECMs from most foreskin fibroblasts), then scarring at the site of incision and implantation may be diminished.

The cells of an Autogenic Living Scaffold may also produce other useful and beneficial components at ratios that more closely simulate or resemble the natural environments that cells are exposed to *in vivo.* This helps the seeded cells to grow and develop into tissue and organs that more closely resemble native tissue and organs. When implanted *in vivo,* the Autogenic Living Scaffold is expected to disappear over time, at least partially, especially if the cells composing the Autogenic Living Scaffold are from a different host. However, the tissue or organ that develops from the cells seeded onto the Autogenic Living Scaffold is expected to remain. Apart from cardiac muscle and the heart, other organs or tissue may be produced using an ALS seeded with cells or tissue of interest. For example, liver tissue may be produced from an ALS that is seeded with hepatocytes; and kidney, pancreas, spinal cord, and other organs and tissues of the body may also be produced by seeding the ALS with the desired cell or tissue type. Autogenic Living Scaffolds seeded with the appropriate cell types may thus be used to grow implantable tissue and organs *in vitro,* for later implantation into an *in vivo* site. In general, when embodiments of the invention comprise the cells and scaffold they produce, the material is referred to as a Living Scaffold or an "Autologous Living Scaffold (ALS)". When the scaffold is seeded with cells to produce a tissue or organ, and used as such, the material is referred to as "ex-vivo cell-produced tissue (ECT) or organ (ECO)".Embodiments of this invention demonstrate that certain cells such as human skin fibroblasts can be induced to produce an extensive 3-D structural filamentous material, referred to herein as cell-produced matrix or cell-derived matrix (CDM), that forms a grid-like or mesh-like matrix which serves to facilitate and aid in the formation of new cell growth and tissue. The resulting Living Tissue Matrix (CDM + the fibroblasts that produced the CDM) is analogous to early connective tissue that forms prior to the migration and population of other cells to form tissues and organs composed of those particular cells. For example, during amphibian embryogenesis, the formation of the heart begins with the formation of this type of fibroblast-populated connective tissue followed by the transdifferentiation of these fibroblasts into cardiac cells to ultimately form the amphibian heart. In embryonic wound healing (and non-scar forming tissue wound healing), the wound bed is first filled with a type of fibroblast-populated connective tissue that is analogous to the Living Tissue Matrix followed by migration and repopulation of the wound site with cells of the damaged tissue. Preliminary experiments have indicated that LTMs provide the scaffolding and/or template for new cell growth for cells of any type (thus serving as "Living Scaffolds"), and serve to aid in cell attachment, migration, proliferation and differentiation, especially tissue generation and regeneration, at sites of tissue damage. Furthermore, the LTM can be transformed into an "implantable blastema" for multi-tissue regeneration *in vitro* by transdifferentiation and dedifferentation of the fibroblasts within the LTM.

The Living Tissue Matrix is produced by the culturing suitable cells such as skin fibroblasts at hyperconfluent density in a completely chemically defined medium (no serum added) such as Matrix Media which causes them to enter a synthetic phase without significant proliferation (prior to being placed under these special conditions, the fibroblasts are induced to proliferate rapidly and are passaged without ever being allowed to attain confluence). In this phase, the secreted extracellular matrix (ECM) differs markedly from that of typical fibroblast cultures and more closely resembles the extracellular matrix found in generating/regenerating environments *in vivo.* There is a high ratio of type III collagen compared to type I along with high concentrations of hyaluronic acid and decorin.

Since the cells produce the entire 3D matrix by themselves, the LTMs appear to more closely resemble *in vivo* generative/regenerative connective tissue than any other matrix, scaffold or structure currently in existence. In fact, LTMs are similar in composition to the type of fibroblast-populated connective tissue that first fills the wound bed in embryonic wound healing (and other non-scar forming tissue wound healing) that regenerates without scarring as opposed to wounds in neonatal or adult mammals that heal with scarring. Furthermore, this method produces a 3-D construct (the LTM) that is significantly thicker and stronger than those obtained using biopolymer gels, such as collagen or fibrin gels. The entire Living Tissue Matrix (cells and ECM) can also be made completely autologous, thus preventing host rejection and making it completely immunocompatible.

The ECM produced in this system provides an optimal environment for de-differentiated or transdifferentiated autologous adult cells within the LTMs to create a regenerative environment and a virtual blastema. In other words, if the millions of fibroblasts within the LTM (the cells that produced the LTM in the first place) are de-differentiated or transdifferentiated, the LTM effectively becomes a structurally sound implantable blastema for multi-tissue type regeneration.

Living Tissue Matrices (LTMs) have potential for being used as soft tissue substitutes since they are produced solely from cells fed with a chemically-defined medium that does not contain animal components. The rapid growth and lack of expensive and inherently variable serum makes the development of LTMs as soft tissue substitutes commercially viable. Due to the relative simplicity of the chemically-defined medium, these cell-produced Living Tissue Matrices can also serve as *in vitro* biological models for the effects of nutritional components and pharmaceutical products on the growth and development of soft tissues. They may also be useful for studying numerous *in vivo* conditions and processes such as wound healing, soft connective tissue formation, fibrosis, and the development and interaction of different cells in a generating/regenerating tissue environment. The greater stability and mechanical integrity of LTMs over both collagen and fibrin gels allows them to retain their structural integrity longer in *in vivo* conditions than reconstituted ECM. The ability to grow LTMs thick and strong in a relatively short period of time in chemically-defined conditions enables the development of an attractive alternative to fibrin gels, collagen gels and even native tissues for tissue replacement.

Many different types of cells may also be seeded in different parts of the Living Scaffold, or they could be sandwiched on top of each other, to produce a variety of ex-vivo cell-produced tissues (ECTs) or organs (ECOs). For example, fibroblasts may first be grown in serum-free conditions favorable to promote a fibroblast ALS. The fibroblast ALS is then seeded with astrocytes, and the serum-free growth conditions (including the media, pH, osmolarity, temperature, oxygen tension, and anything else required) are adjusted to be favorable to the growth of the astrocytes. If needed, other components are added to keep the ALS alive and healthy. This entire structure or system may also be grown on porous membranes, such as TransWells™ or BD Falcon™/BioCoat™ Cell Culture Inserts, which allows media to be added to both the basolateral and apical sides of the ECT complex. Thus, media components more favorable to the growth and survival of the original cells (for example, fibroblasts) of the ALS may be added to the basolateral side of the entire ECT system or complex, while media components more favorable to the growth of the cells and tissue that are seeded onto the ALS may be added to the apical side of the entire system. Another option is to use cell culture systems such as OptiCell™ that allows gas exchange to occur across the walls of the cell culture vessel where the cells can attach and grow, thus allowing an ALS or LTM grown up to a thickness of about 2mm to be developed *in vitro.* Once a favorable layer of astrocytes has grown on the layer of fibroblasts, they may both be referred to as together making up the ECT, or the fibroblasts alone may be referred to as the LTM. Nerve cells may then be seeded on top of the layer of astrocytes, and the serum-free growth conditions again adjusted to be favorable to the growth and development of the nerve cells. If needed, other components are again added to keep the ALS and astrocytes alive and healthy, and the entire system may also be grown on a porous membrane to allow different media components to be added to the apical and basolateral sides of the entire system or ECT complex, as described above. Additional layers, such as skeletal muscle myocytes that might form into skeletal muscle tissue that is innervated by the already seeded nerves, may continue to be added in this or a similar fashion, as desired.

In another embodiment, the ALS or LTM may also be grown into specific shapes by molds, and may also be reshaped to some degree. For example, a sheet of the above example of an ALS, seeded first with astrocytes and then nerve cells, may be rolled into a cylinder. This cylinder may then be implanted into a spinal cord *in vivo.* The ALS also provides mechanical support to the seeded cells. For example, in a particular embodiment, an ALS seeded with neurons may be mechanically stressed and compressed, without major damage to the neurons, even though such a degree of mechanical stress and compression kills most neurons when grown in the absence of an ALS or without formation of the ECT. The ALS of other embodiments may also be introduced to mechanical stress or tension which may change the properties of the ALS and any cells or tissue that are growing on it.

In one particular embodiment, the fibers of a Fibroblast Autogenic Living Scaffold may also be made to grow in parallel, which helps seeded nerve cells to also grow in parallel along these fibers, especially when Schwann cells are previously seeded onto the scaffolds and first start growing in parallel along these fibers. This may be even more useful when implanted in the spinal cord, since the implanted nerve cells may then be aligned in the same direction as the native nerve cells in the spinal cord. In still another embodiment, a sheet of ALS with the seeded neurons may also be rolled into a cylinder prior to implantation to produce a structure with layers of neurons aligned in the same general direction as the native neurons in the spinal cord. Similar things may be done for implantation into other tissues and organs.

In other particular embodiments, cell to cell, tissue to tissue, and tissue to cell interactions may also be studied *in vitro* and *in vivo* with Autogenic Living Scaffolds and LTMs, such as by sandwiching different cells. In yet another embodiment, Autogenic Living Scaffolds and LTMs may be used as *in vitro* biological models for studying the growth and development of cells, tissues, organs, systems, diseases, and different responses in organisms. For example, the wound response (in which fibroblasts play an active role) on different types of cells and tissues may be studied *in vitro* by using this technology.

A current shortage of load-bearing tissue has created a demand for human-made tissues that can withstand *in vivo* mechanical forces Most of the strength of connective tissues is due to the collagen content per unit mass of tissue. The high tensile strength of collagen is largely attributable to the presence of intermolecular covalent cross-links between the collagen fibrils Strength increases with increasing collagen fibril diameter (which are typically in the order of up to 100 nm in diameter in native tissue, and 40-60 nm in developing native connective tissue), as well as with increasing density and crosslinking of collagen fibrils. For the collagen in fibroblast based ALSs (fALS) and LTMs, a density of over 100 collagen fibrils/$\mu$m$^2$ can be achieved with a high degree of cross-linking between the fibrils. The collagen fibril diameters in ALSs where the fibroblasts are derived from the dermis of neonatal human foreskin, have so far been in the order of 40-50 nm, indicating similarity to young connective tissue *in vivo* that has greater regenerative potential than mature connective tissue. Mechanical strength of collagen can also be increased through magnetic alignment of the collagen fibrils and by the addition of lysyl oxidase that induces additional cross-links between the collagen fibrils Strength also increases with the addition of fibronectin, which in turn increases actin organization and regulates the composition of the ECM. For example, fibronectin induces a 5-fold increase in the ultimate tensile strength of fibroblast populated collagen lattices In particular embodiments, fibronectin is generally the second most abundant ECM protein after collagen in ALSs fed with the Matrix Media.

Fibroblasts (especially foreskin fibroblasts) secrete numerous growth factors including nerve growth factor (NGF), brain-derived neurotrophic factor (BDNF), and neurotrophin-3 (NT-3), as well as fibroblast growth factor (FGF), and platelet-derived growth factor (PDGF), all of which promote neuron regeneration and survival. The embodiments of ALSs and LTMs described herein more closely mimic the extracellular environment that nerve cells are normally exposed to *in vivo* than any other currently available scaffolds, and even allow primary nerve cells to form active 3-D neural networks *in vitro* that can serve as *in vitro* 3-D models for potential therapeutic agents for neuronal regeneration, as may also be used to functionally replace injured spinal cord neurons *in vivo.*

Previous experiments show that fibroblasts grafts obtained from an *in vivo* site have allowed axons to elongate ~0.5mm/month at a density of -28 axons/$\mu$m$^2$, and have provided functional recovery within 3 months of up to ~10 on the BBB scale (Basso, Beattie and Bresnahan locomotor rating scale - scale range: 0-21, with 0 representing no function, and 21 representing complete locomotor functionality - in animals; (Lu *et al.,* (2002) *Brain* 125(Pt 1):14-21), when implanted into chronically injured spinal cord in rats and monkeys. When the fibroblasts are genetically modified to express 10x more BDNF and NGF, these numbers jump to ~2.1mm/month at a density of -70 axons/$\mu$m$^2$, and functional recovery is further slightly improved. The fibroblasts stopped proliferating after they were implanted at high density, and the grafts also prevented the formation of fibrosis at the site of injury and implantation. Furthermore, the fibroblast grafts promoted rapid and extensive migration of Schwann cells into the grafts from the peripheral nervous system, which is considered to be a major and necessary factor of functional spinal cord regeneration.

Other research showed that collagen gels and collagen filament implants allowed axons to elongate up to 5 mm within a month, and provided functional recovery within 3 months of up to ~12 on the BBB scale when implanted into injured spinal cords in rats. Still others have shown that fibronectin mats have allowed axons to elongate up to 4-5 mm and reach diameters of up to 3.5 $\mu$m within a month, and provided functional recovery within 3 months of up to ~10 on the BBB scale when implanted into injured spinal cords in rats. Implanted fibronectin has also been found to aid in the proper orientation of elongating neuronal axons at the site of implantation in the damaged spinal cord, as well as in attracting Schwann cells from the peripheral nervous system.

In the case of particular embodiments of fALS of the present specification, the high density of fibroblasts along with the insoluble ECM proteins collagen and fibronectin of the ALS work in conjunction to promote axon elongation and functional recovery when implanted into chronically injured spinal cord. It is within the realm of the presently disclosed ALS systems that a particular ALS seeded with primary neuronal cells that produces differentiated nerve cells and/or tissue may allow axons to elongate up to as much as ~10mm/month and may promote the beginnings of functional recovery within 1 month, ideally increasing to a value approaching 20 on the BBB scale after 3 months. It is also within the realm of embodiments of the presently disclosed ALS systems that a particular ALS seeded with primary neuronal cells that produces differentiated nerve cells and/or tissue may allow between ~0.5 mm/month axon elongation and about 10 on the BBB scale within 3 months, and up to about 10mm/month axon elongation and preliminary functional recovery within 1 month, increasing to a value approaching 20 on the BBB scale after 3 months. Other particular embodiments of fALS systems seeded with primary neuronal cells are envisioned to allow intermediate axon elongation, anywhere from about 3-8 mm/month of axon elongation, with functional recovery again approaching about 20 on the BBB scale after 3 months.

It is known that implanted neural progenitor cells survive for long periods of time at the site of implantation in injured spinal cord and support axonal elongation and limited functional recovery, and that neural progenitor cells differentiate into nerve and glial lineages and survive for much longer than neuroblastomas (which usually die before making any active synaptic connections - a requirement for survival and nutritional support from the glial cells). Neural progenitor cells are also deemed far more safe and predictive than stem cells, which are difficult to control and are thought to be one of the main promoters of cancer in the central nervous system. Unfortunately, neural progenitor cells differentiate only into astro- and oligodendro-glial lineages when implanted into injured spinal cord, or when cultured *in vitro* in the absence of ALS systems according to the present invention. When neural progenitor cells are seeded *in vitro* onto an ALS in accordance with embodiments of the present invention, however, the neural progenitor cells differentiate into nerve and glial lineages, as shown in Figures 1 through 7.

Furthermore, neuroblastomas seeded *in vitro* together with neural progenitor cells or astrocytes onto ALS according to embodiments herein, are envisioned to allow long-term differentiation and survival of the neuroblastomas on the ALS and also at the site of *in vivo* implantation in injured spinal cord. Also, basic fibroblast growth factor, which is secreted by the fibroblasts of particular embodiments of the ALS systems described herein; induces the secretion of neural growth factor (NGF) by astrocytes grown on the ALS in serum-free culture conditions.

**Research Design and Methods**

Example: Using the ALS as a scaffold for creating a nerve graft:

There is currently no proper treatment for spinal cord injury. Limited spinal cord regeneration has been achieved in a small number of patients through physical rehabilitation and training, peripheral nerve grafts, and by transplanting fetal spinal cord tissue. Most patients are limited to being dependent on the use of a wheelchair and even on devices that sustain/replace lost autonomic function.

There are a number of approaches in current research into treatments for spinal cord injury. The current limitation of all these approaches is that they restore only partial and generally minimal functionality to the injured spinal cord. The first clinical trials for the treatment of spinal cord injury began on July 11, 2002, in Australia by transplanting olfactory ensheathing glia into patients' spinal cords. The limitation of this approach is that it takes several years to restore some of the functions lost due to the spinal cord injury, and the restoration of these functions is limited (from a 1 to 4.3 on the Basso, Beattie and Bresnahan (BBB) locomotor rating scale (scale range: 1-20, with 1 representing no function, and 20 representing complete locomotor functionality) in animals). Another clinical trial involving the vaccination of Copolymer-1 into patients with recent (<14 days) spinal cord injury is about to start in Israel and the United States, but this treatment is designed more to slow down and possibly reverse the degeneration of nerve cells in diseases such as Parkinson's disease rather than being designed as a means for regenerating injured spinal cord nerve cells.

There is somewhat of a consensus in the field of neuroregeneration that the necessary elements for potential tissue engineered nerve constructs include: 1) a scaffold for nerve growth and axonal migration; 2) support cells; 3) growth factors; and 4) extracellular matrix. To date, no system that includes all the elements has been described or disclosed that successfully generates nerve cells *in vitro,* for later *in vivo* implantation. In addition, there are no *in vitro* 3-D models of neuronal growth or regeneration. Since the ALPS provides an environment that closely resembles the extracellular environment that neurons are normally exposed *to in vivo,* the proposed tissue engineered nerve grafts created using the ALS could be used for a variety of *in vitro* applications, such as studying the effects of potential therapeutic agents on neuronal regeneration. In terms of serving as a nerve graft for replacing damaged spinal cord, this system uses only human components and has the potential to replace/regenerate spinal cord that is extensively damaged over large areas and distances since it contains its own nerve cells/neural tissue.

Preliminary studies have indicated that the majority of neurons grown within an ALS in accordance with the present invention are still alive after a ~200-$\mu$m thick graft has been gently folded into itself and then gently unfolded, as determined by a neuronal viability assay (Molecular Probes LIVE/DEAD Viability/Cytotoxicity kit # L-3224) before and after handling of the nerve graft (data not shown). However, in such an ALS, the neurons represent only a small fraction of the ~200-$\mu$m thick graft. Thus neurons may be seeded onto thinner supportive ALSs wherein the ultimate tensile strength (UTS) of the ALS is increased by small modification to the ALS Medium. Alternatively, in other embodiments the porosity of the ALS may also be increased, to allow neurons to migrate throughout thicker ALSs. The fALSs in accordance with particular embodiments of the present invention are prepared by seeding human foreskin fibroblasts at high density onto wells or TransWells™, and allowing the fibroblasts to develop a 3-D matrix having a thickness of between 50 to several hundred micrometers over a period of several weeks. The fibroblasts are fed with a defined "chemical" media that does not contain any serum or animal components, referred to herein as Fibroblast Autogenic Living Scaffold Medium (fALS Medium), an example of which is Matrix Media, and described in Example 1. In a particular embodiment, after the 3-D matrix (ALS) is produced, the ALS is seeded with primary neuronal cells. The ALS with the seeded neuronal cells is then fed with another defined "chemical" medium (again, a medium with no serum or animal components) that supports the growth of the neurons and retards the growth of the fibroblasts. In one embodiment, the ALS seeded with primary neuronal cells is maintained in TransWells™, allowing the basal side of the ALS to continue being fed with the first media (fALS Media) while the apical side of the ALS is fed with the second media.

The development of a functional neural network *in vitro* allows the nerve graft to have more utility when implanted, and is important for studying the effect of different pharmacological agents and methods on the *in vitro* 3-D neural network model disclosed herein. To date, no functional neural networks grown *in vitro* have been able to be transferred and implanted into a host due to the frailty of these neural networks - nerves are so frail that they cannot even support their own weight, and no scaffolds (apart from the fALS disclosed herein) have yet been developed that provide adequate structural support to these nerves. To optimize the production of functional neural networks, the nerve graft in one embodiment may be grown on a 64-microelectrode array, or alternatively, voltage-sensitive dyes may be used to determine the functionality (presence of action potentials) and connectivity (presence of phasic bursting patterns) of the neurons (see Mistry et al., (2002) Proc Natl Acad Sci USA 99(3):1621-6 and Segev et al., (2003) Phys Rev Lett 90(16): 168101) in the ALS. In this way different types of primary neurons (such as neural progenitor cells and neuroblastomas) may be tested to determine which types differentiate and produce the most favorable functional neural networks in the particular embodiments of ALS as described herein.

In other embodiments the effects of different nutritional supplements and growth factors on the development of the functional neural networks in the ALS may also be studied in this way. For example, FGF-2 and BDNF have been shown to support the development of extensive and spontaneously active neural networks from primary neurons within 3 weeks (see Mistry *et al.,* 2002). Preliminary results with ALSs seeded with primary neuronal cells in accordance with the present invention show that the carbohydrate source galactose is favored over glucose by neurons, and that galactose also significantly retards the growth of the fibroblasts in the ALS. Thus, in embodiments of the present invention, once the neurons are seeded onto the ALS, the growth media is changed from one that supports ALS growth to one that promotes neuronal growth and differentiation, while at the same time retards the growth of fibroblasts. This prevents the fibroblasts from over-running the neurons and effectively preventing neuronal development. In other embodiments, the fibroblasts may be genetically engineered to secrete more growth factors such as NGF, BDNF, FGF-2, and bFGF to enhance neuron survival and development even further.

Specific neuronal markers such as Anti-Hu MAb 16A11 (Marusich et al., (1994) J. Neurobiol 25(2): 143-155 may be used to differentiate neurons from fibroblasts (see Figures 3 through 7, and 12 through 13). The degree of attachment and differentiation of neurons on ALS can be determined (no. of differentiated neurons/no. of primary neurons seeded), along with how far the axons elongate through the ALS (average length of axons in 4-10 fields of view (see Segev et *al.,* 2003)) and the length of time that the neurons are viable on the ALS (from the time of seeding). Determining the length of time that the neurons are viable on the ALS allows the graft to be implanted days prior to the degeneration of the nerves, thereby ensuring that the nerves remain alive and functional in the host/patient to better the chances for neurogenesis. In addition, histology and transmission electron microscopy (tEM) may be used to observe the arrangement and connectivity of the neurons (Jin et al., (2002) Exp Neurol 177(1):265-75 and Tuszynski et al., (2002) J Comp Neurol 449(1):88-101 in the ALS.

It is expected that superior results may be achieved when a particular embodiment of an ALS nerve graft is implanted 2 weeks after spinal cord injury, since this is when BDNF-expressing and supporting microglia, as well as peri-wound sprouting around the site of injury, begin to reach significant levels. However, in particular embodiments, the ALS nerve graft contains active neurons, including primary neurons that have begun to differentiate and form active neural connections (see Figures 2-4, for example), so positive results are expected regardless of when the ALS nerve graft is implanted.

In other embodiments, the ALS nerve graft has the flexibility of taking on almost any non-rigid shape and may be rolled up into a ball or cylinder. Several thin nerve grafts may also be layered on top of each other to form different parallel layers of neural networks, which in turn may again be rolled up into a cylinder or formed into some other shape. Alternative forms and structures of ALS nerve grafts are expected to give better results in terms of functional recovery for each type of spinal cord injury (longitudinal, transverse, shear, etc.). The requirement for immune-suppressants may also be determined, although such agents are not expected to be required since nerve cells are not attacked by the immune system in humans and fibroblasts do not elicit much of an immune response. Alternatively, in other particular embodiments nude rats may be used as models for implanting human nerve grafts, to study functional recovery promoted by the ALS nerve grafts in accordance with embodiments of the present invention.

In still other embodiments, the ALS nerve grafts as herein described, may be benchmarked against other current approaches, e.g., according to the extent of functional recovery in spinal cord injury-induced animals (using the BBB scale as a standard), or according to the extent of active functionality of implanted/regenerated nerve cells (as determined electrophysiologically), or according to the extent of complications, the time to functional recovery from the date when the procedure is started, and from a cost/feasibility perspective.

## EXAMPLES

*Example 1a - Serum-Free Chemically-Defined Medium for Growing Fibroblast ALS - "fALSMedium"or "Matrix Media"*

A chemically defined media formulation in accordance with the present invention contains:

A 3:1 ratio of DMEM (high glucose (4.5g/L); with L-glutamine and sodium pyruvate) and Ham's F12 medium supplemented with the following components:

- 4.2 x $10^{-10}$M Epidermal Growth Factor (in human serum albumin)
- 2.8 x $10^{10}$M Basic Fibroblast Growth Factor

- 8.6 x $10^{-5}$M insulin
- 1.0 x $10^{-7}$M dexamethasone
- 3.2 x $10^4$M L-ascorbic acid phosphate magnesium salt n-hydrate
- 2 x $10^{10}$M L-3,3',5-triiodothyronine
- $10^{-4}$M ethanolamine
- 3.9 x $10^{-8}$M selenious acid
- 4 x $10^{-3}$M Glutamax™
- 3.3 x $10^{-6}$M glutathione (reduced)
- 1% penicillin/streptomycin/amphotericin B

In addition, other embodiments and variations of the above-listed medium may contain additional components, such as any one or more of the following components:

- Platelet derived growth factor (PDGF)
- 100:68 ratio of glycine : L-proline
- L-cysteine
- Trolox

Also described herein is a chemically defined media formulation as above, wherein concentrations may vary as required, as long as the total osmolarity in the medium is kept at acceptable levels for growth of the ALS. For example, L-ascorbic acid phosphate magnesium salt n-hydrate may range in concentration from 0.1 mM to 3 mM; EGF may range in concentration from 0.002 nM to 2 nM; bFGF may range in concentration from 0.03 nM to over 3 nM; insulin may range in concentration from 10 $\mu$M to 1000 pM; PDGF may range in concentration from 0.1 ng/ml to 10 ng/ml; L-3,3',5-triiodothyronine may range in concentration from 0.1 nM to 10 nM; ethanolamine may range in concentration from 1 $\mu$M to 10,000 $\mu$M; selenious acid may range in concentration from 10 nM to 1000 nM; Glutamax™ may range in concentration from 0 mM to 10 mM; glycine:L-proline concentrations (still at a 100:68 ratio) may be supplemented with from 0 mM glycine: 146 mM L-proline; to 2.675 mM glycine: 1.965 mM L-proline; dexamethasone concentrations may vary from 1 nM to 1000 nM; L-cysteine concentrations may be supplemented with additional 0.1 mM to 1 mM.

*Example 1b - Serum-Free Chemically-Defined Medium for Growing Neuronal Cells/Tissue - "Neural Medium"*

A chemically defined media formulation contains:

A 2:3 ratio of DMEM/F12 and Neurobasal Medium (see Gibco-Invitrogen Corporation at www.invitrogen.com, or www.invitrogen.co.jp/focus/161006.pdf) supplemented with the following components:

- 3 x $10^{10}$M Fibroblast Growth Factor 2
- 8.5 x $10^{-6}$M D(+)galactose
- 6.0 x $10^{-8}$M progesterone
- 6.0 x $10^{-7}$M retinyl acetate
- 9.0 x $10^{-8}$M corticosterone
- 1.0 x $10^{-4}$M putrescine
- 1.0 x $10^{-5}$M carnitine
- 1.3 x $10^{-5}$M linoleic Acid
- 4.3 x $10^{-6}$M linolenic Acid
- 4.0 x $10^{-6}$M biotin
- 4.0 x $10^{-6}$M Trolox
- 1% penicilin/streptomycin/amphotericin B

In addition, other variations of the above-listed medium may contain additional components, and concentrations may vary as required.

*Example 2. Production of Extracellular Matrix by Fibroblast Cells isolated from the dermis of newborn human foreskin*

Large numbers of fibroblast cells were isolated from the dermis of newborn human foreskin. Cells were proliferated in cell culture flasks fed with DMEM Medium with 10% Nu-Serum (or FBS or BCS) for several weeks. After a few passages, the fibroblast cells were centrifuged at 1000 RPMs, the supernatant decanted, and the cells pooled. These pooled fibroblasts cells were resuspended in ALS Medium as described above in Example 1, then seeded into TransWells™ or BD Falcon™/BioCoat™ Cell Culture Inserts or 6- or 12-well plates or other small containers suitable for *in vitro* cell culture, at superconfluent conditions, whereby cell density was between about 200,000 to greater than about 1,000,000 cells/cm$^2$. The fibroblast cells were maintained at hyperconfluent conditions for about 3 weeks (or optionally about 1 week or longer than 10 weeks), during which time they were observed to produce Extracellular Matrix, thus creating a Fibroblast ALS about 300 $\mu$m thick. See Figs. 3-9, and Figs.12-13, for example, showing clearly visible extracellular matrices. Extracellular Matrices that have been produced by the fibroblasts result in ALSs that are 50 $\mu$m to 500 $\mu$m thick, or even up to or greater than 1 mm thick.

In one embodiment, the fibroblast ALS is grown on a circular porous polyethylene (Huang et al., (1993) Annal Biomed Eng 21(3): 289-305 and Uysal et al., (2003) Plast Reconstr Surg 112(2):540-6) or ceramic support that exposes both sides of the ALS to media. This allows the ALS to grow faster and thicker due to the shorter diffusion distance for gases and nutrients than when the ALS is exposed to media on only one side at the bottom of a culture well. Alternatively, the ALS is grown on 0.4$\mu$m TransWells™ (Corning, 2003), although this limits the subsequent seeding of other cell types to just one side of the CDM. The use of OptiCells™ allows the ALS to grow up to about 2 mm thick *in vitro* due to the efficient gas exchange from both sides of the ALPS. Another alternative is to use MINUCELLS™ and MINUTISSUE™ since they allow the constant perfusion of media and oxygen to the cells and tissue of/on the ALS. Using 0.4$\mu$m TransWells™ or center-well organ culture dishes (BD Biosciences # 353037) also allows more nutrient media to be used and permits the cells to be closer to an $O_2$ source. The availability of $O_2$ may be further enhanced by increasing the partial pressure of $O_2$ (p$O_2$) that the cells and inside of the culture vessel are exposed to. The pores in the porous polyethylene or ceramic support provide adequate sites of ECM attachment for the ALS. Cells are prevented from growing on the wells and the outside circle of the porous polyethylene or ceramic supports by treating these areas with a non-binding substance such as SigmaCote (Sigma # SL-2), once the ALS is grown in a suspended or floating state. Suspended ALSs may be induced to have more dendritic fibroblast morphology, thereby allowing more flexibility in modifying the characteristics of the ALS, for example, through the use of mechanical stimulation.

*Example 3. In vitro Protocol for Generating Neuronal Cells/Tissue*

A large number fibroblast cells (Cambrex CC-2509) were centrifuged, at approximately 1000 rpm for about 8min, followed by removal of the supernatant. The resulting fibroblasts were then resuspended in ALS Medium and seeded into TransWells™ or BD Falcon™/BioCoat™ Cell Culture Inserts at superconfluency (between about 1,000 cells/mm$^3$ to greater than 200,000 cells/mm$^3$), and fed with ALS Medium every 2-3 days for about 2-3 weeks or longer at hyperconfluent conditions until the desired amount and thickness of ALS had formed.

At this point, the ALS containing fibroblasts and extensive CDM was seeded with neural progenitor cells (Cambrex CC-2599), and the basolateral side of the TransWells™ continued to be fed with fALS Medium while the apical side was fed with Neural Medium every 2-3 days for another 2 - 4 weeks or longer.

With time and appropriate growth factors, secreted by the fibroblasts in response to the presence of the seeded neural progenitor cells and so present in the ALS (but they may also be added as a supplement), the neural progenitor cells began to differentiate into both nerve cells, as evidenced by formation of nerve axons, and glial cells. Neural progenitor cells implanted into mammalian spinal cord and grown in the absence of an ALS system do not differentiate into both nerve cells and glial cells; they only differentiate into glial cells. But as can be seen in Figure 2, neural progenitor cells, grown on a fibroblast ALS, have differentiated into both nerve axons and glial cells. Additionally, Figure 1 shows methylene blue staining of neurons that have differentiated from neural progenitor cells grown on a fibroblast ALS.

Figures 3 through 7 show sections of fibroblast ALS systems containing neuronal cells/tissue that are expressing anti-Hu MAb 16A11, an early marker of vertebrate neurogenesis that is expressed shortly after neuronal terminal mitosis. The marker is observed in nerve cell bodies as reddish brown spots; the cell nuclei are blue. As can be seen, varying-sized regions (different cross-sections of nerve cell bodies) expressing the anti-Hu MAb 16A11 marker are indicated, ranging in size from 4 $\mu$m to over 20 $\mu$m.

*Example 4. In vitro Protocol for Generating Muscle Cells/Tissue*

A large number fibroblast cells (Cambrex CC-2509) were centrifuged, at approximately 1000 rpm for about 8min, followed by removal of the supernatant. The resulting fibroblasts were then resuspended in fALS Medium and seeded into TransWells™ or BD Falcon™/BioCoat™ Cell Culture Inserts at superconfluency (between about 1,000 cells/mm$^3$ to greater than 200,000 cells/mm$^3$), and fed with fALS Medium every 2-3 days for about 2-3 weeks or longer at hyperconfluent conditions until the desired amount and thickness of ALS had formed.

At this point, the ALS containing fibroblasts and extensive CDM was seeded with human skeletal muscle myoblasts (Cambrex CC-2580), and the basolateral side of the Trans Wells continued to be fed with ALS Medium while the apical side was fed with Skeletal Muscle Medium (Cambrex CC-3160) every 2-3 days for another 2 - 4 weeks or longer. With time and appropriate growth factors, secreted by the fibroblasts in response to the presence of the seeded skeletal muscle myoblasts and so present in the ALS (but they may also be added as a supplement), the skeletal muscle myoblasts began to differentiate into skeletal muscle cells. Figures 12 and 13 show trichrome staining of two different sections of a fibroblast ALS system containing muscle cells/tissue, one about 70 $\mu$m thick (Fig. 12) and the other section about 115 $\mu$m thick (Fig. 13). The fibroblast extracellular matrix of collagen fibers are blue; the nuclei of the ALS fibroblast cells are (bluish) black; and the young skeletal muscle cells within the ALS are observed as reddish spots within the ALS sections.

### Example 5. In vitro Protocol for Generating Liver Tissue

A large number fibroblasts cells (Cambrex CC-2509) were centrifuged, at approximately 1000 rpm for about 8min, followed by removal of the supernatant. The resulting fibroblasts were then resuspended in fALS Medium and seeded into TransWells™ or BD Falcon™/BioCoat™ Cell Culture Inserts at superconfluency (between about 1,000 cells/mm$^3$ to greater than 200,000 cells/mm$^3$), and fed with fALS Medium every 2-3 days for about 2-3 weeks or longer at hyperconfluent conditions until the desired amount and thickness of ALS had formed.

At this point, the ALS containing fibroblasts and extensive CDM was seeded with human hepatocytes (Cambrex CC-2591), and the basolateral side of the TransWells continued to be fed with fALS Medium while the apical side was fed with Hepatocyte Medium (Cambrex CC-3198) every 2-3 days for another 2-4 weeks or longer. With time and appropriate growth factors, secreted by the fibroblasts in response to the presence of the seeded hepatocytes and so present in the ALS (but they can also be added as a supplement), the hepatocytes began to differentiate into albumin-secreting liver cells and tissue.

### Example 6a. In vivo Protocol for Generating/Regenerating Neuronal Tissue Using In Vitro ALS Systems Seeded with Neural Progenitor Cells Rat/Rodent Spinal Cord

The lower spinal cord of an adult nude Fisher rat is partially severed under general anesthesia (a complete and full 5mm long spinal cord transection) such that the rat is paralyzed from below the point of injury (the site of injury is chosen such that the lower limbs are non-functional after excision - generally T9-T10). Neuronal cells/tissue generated *in vitro* within a fibroblast ALS system seeded with human neural progenitor cells or neuroblastomas or neuronal stem cells or other primary neural cell line, such as detailed in Example 3, are next implanted into the rat spinal cord at the site of injury.

The fibroblasts within the ALS begin attaching to the site of implantation immediately, and continue attaching for days and weeks, as the neuronal cells grow and form active neural connections with existing nerves on each side of the implantation site. Any remaining primary neurons at the time of implantation appear to differentiate into functional nerve cells and tissue. The rat is maintained and observed for 8 weeks or longer, and observed for indications of neurogenesis, such as the ability to move its hind legs, or even the ability to walk with erratic use of its hind legs, or the ability to walk using its hind legs for a short a distance. Experiments have so far resulted in experimental animals regaining sensation and movement corresponding to up to 14 on the BBB scale, as compared to an average of 2 on the BBB scale for control animals.

### 6b. In vivo Protocol for Generating/Regenerating Neuronal Tissue Using In Vitro LTM Systems where the fibroblasts within the LTM are Transdifferentiated into Neurons Rat/Rodent Spinal Cord

The lower spinal cord of an adult nude Fisher rat is partially severed under general anesthesia (a complete and full 5mm long spinal cord transection) such that the rat is paralyzed from below the point of injury (the site of injury is chosen such that the lower limbs are non-functional after excision - generally T9-T10). Neuronal cells/tissue generated *in vitro* within an LTM by transdifferentiating the fibroblasts within the LTM into neurons (by placing the LTM in Neurogen media with cytochalasin B for 60h, followed by replacing the media with fresh Neurogen media for 2-3 days) are next implanted into the rat spinal cord at the site of injury.

The neuronal cells within the LTM grow and form active neural connections with existing nerves on each side of the implantation site over several weeks. Remaining neurons on each side of the transection site appear to also start growing into the LTM over several weeks. The rat is maintained and observed for 8 weeks or longer, and observed for indications of neurogenesis, such as the ability to move its hind legs, or even the ability to walk with erratic use of its hind legs, or the ability to walk using its hind legs for a short a distance. Experiments have so far resulted in experimental animals regaining sensation and movement corresponding to up to 16 on the BBB scale, as compared to an average of 2 on the BBB scale for control animals.

*6c. In vivo Protocol for Treating Spinal Cord Regeneration in an Animal*

Fibroblast cells from human foreskin are cultured to produce an ALS or LTM system having an extensive ECM, as detailed in Example 2. The ALS is then seeded with neural progenitor cells or neuroblastomas or neuronal stem cells or other primary neural cell line as described in Example 3, or the fibroblasts within the LTM are transdifferentiated into neurons as in example 6b, until nerve cells and/or tissue are evident. The ALS plus primary neural cells/tissue or neuronal LTM is next implanted into the animal's spinal cord near or at the site of degeneration, or at several sites along the spinal cord if degeneration is pervasive.

The treated animal is then maintained and observed for 8 weeks or longer, as required, while monitoring for evidence of spinal cord regeneration, such as new-found movement, feeling and sensation. Several treatments with neuronal LTM or ALS systems having primary neural cells and/or tissue may be performed, to increase the spinal cord regeneration seen, depending upon the severity of degeneration in the animal to be treated.

Such a procedure may be used on animals, including humans, suffering from a wide-spectrum of neurodegenerative diseases such as Lou Gehrig's disease, Huntington's disease, spinal cord compression due to crushed vertebrae, spinal cord severance, etc.

*7. In vivo Protocol for Generating/Regenerating Cartilage Tissue Using In Vitro ALS Systems Seeded with Chondrocytes*

A large number fibroblast cells were centrifuged, at approximately 1000 rpm for about 8min, followed by removal of the supernatant. The resulting fibroblasts were then resuspended in ALS Medium and seeded into TransWells™ or BD Falcon™/BioCoat™ Cell Culture Inserts at superconfluency (between about 1,000 cells/mm$^3$ to greater than 200,000 cells/mm$^3$), and fed with ALS Medium every 2-3 days for about 2-3 weeks or longer at hyperconfluent conditions until the desired amount and thickness of ALS had formed. The ALS containing fibroblasts and extensive CDM was then seeded with chondrocytes (Cambrex CC-2550), and the basolateral side of the TransWells™ continued to be fed with ALS Medium while the apical side was fed with Chondrocyte Medium (Cambrex CC-3216) every 2-3 days for another 4-6 weeks or longer. With time and appropriate growth factors, secreted by the fibroblasts in response to the present of the seeded chondrocytes and so present in the ALS (but supplemental factors can also be added as required or desired), the chondrocytes began to differentiate into cartilage tissue.

The Autogenic Living Scaffold may also be first grown into specific shapes by molds, and may also be reshaped to some degree. For example, a "balloon" or hollow disc of a Fibroblast Autogenic Living Scaffold may be prepared by growing the fALS culture around a Teflon disc or sphere with a thin coating of an easily degradable protein or other substance and then slitting one side to remove the disc or sphere and thereby creating the hollow space or "balloon" shape. After preparation of the ALS "mold", it may be filled with chondrocytes in a gel (such as an alginate gel) that may be placed/injected into the open inner space of the ALS disc or balloon mold, and then closed back up to create dense, compact cartilage tissue over time. The ALS allows nutrients and gases to pass through it to the chondrocytes, while at the same time retaining most of the signaling molecules secreted by the chondrocytes and secreting certain growth factors and substances that promotes the formation of functional cartilage tissue. The high tensile strength of the Fibroblast ALS also creates mechanical stresses onto the chondrocytes and newly formed cartilage that promotes the development of functionally strong cartilage. The outside layer of ALS around the formed cartilage disc also promotes the attachment and integration of the cartilage disc once it is implanted. Such a procedure may be used to create, for example, cartilage discs for replacement within a vertebrate spinal column.

Any desired shape or size may be made by creating the appropriate ALS mold, and the resulting cartilage tissue formed within the ALS mold may be used to treat an animal, including a human, suffering from cartilage damage. The same is true of bone damage, where osteoblasts are used instead. In such treatments, the ALS mold plus interior seeded connective tissue cells, or plus interior cartilage tissue, is placed/inserted into the animal's joint, or placed/inserted near or at the site of injury where cartilage regeneration is desired, or at several sites.

The treated animal is then maintained and observed for several weeks and months or longer, as required, while monitoring for evidence of cartilage regeneration by using MRI imaging or other soft-tissue detection techniques, and by observation of physical indications such as joint function, levels of pain, strength, flexibility, etc. Several treatments with ALS molds and seeded chondrocytes may be performed, to increase the cartilage regeneration seen, depending upon the severity of the injury or cartilage degeneration in the animal to be treated.

Such a procedure may be used on animals, including humans, suffering from a wide-spectrum of cartilage degeneration conditions such as cartilage degradation/degeneration in the joints, including knees, ankles and fingers, in the spinal column, and in the nose, ear, throat, and elsewhere.

*8. Living Tissue Matrix (LTM)*

A strong and thick cell-produced Living Tissue Matrix (LTM) can be produced within three weeks, making it a viable and attractive alternative to reconstituted gels, such as cell-populated fibrin or collagen gels. Data from comparative studies show that a completely cell-produced ECM is mechanically superior to reconstituted ECM, and more closely resembles native *in vivo* ECM than reconstituted ECM. This strength is highly correlated to the fraction of very stable collagen. In addition to closely approximating native generating/regenerating tissue, LTMs also support significantly faster rates of cell adhesion, migration, proliferation, differentiation, and acquisition of *in vivo*-like morphology than reconstituted ECM. The extracellular matrix (ECM) portion of the LTMs consists of several components such as collagen I, collagen III, fibronectin, proteoglycans, sulfated glycosaminoglycans, hyaluronic acid, and decorin.

Extensive preliminary studies have indicated that these matrices are composed of numerous extracellular matrix proteins in amounts, ratios and arrangement that more closely resemble young, native, blastema-like tissue than any other matrices or scaffolds currently in existence. These matrices are embedded with multi-layered fibroblasts that created the LTM in the fust place. Dedifferentiating these multi-layered fibroblasts in the matrix could essentially lead to a blastema-like structure that can be implanted onto the site of tissue injury for restoration of a functional multi-tissue type structure. Transdifferentiating some or all of these multi-layered fibroblasts in the matrix could then aid the blastema-like structure so formed to start developing and differentiating along a more controlled pathway.

Figure 14 shows an H&E stained cross-section of early nerve differentiation on cell-produced Living Tissue Matrices. Human neural cell bodies having Hu16A11 markers (a marker of early neurogenesis) as visible, with the stained markers discernable as dark (brown) stained regions in the neural cell bodies.

9. Genetically Engineering fibroblasts to make a stronger and thicker ALS or LTM.

A human gene coding for fibroblast growth factor 2 (FGF-2) was inserted into the genome of human neonatal foreskin fibroblasts (obtained from the American Tyoe Culture Collection (ATCC, Manassas, VA)) under the regulation of a β-actin promoter. These transformed or genetically engineered fibroblasts, produced a significantly stronger and thicker cell-derived matrix than control fibroblast cultured for a 3-week period according to the methods of example 2. The genetically engineered fibroblasts were found to secrete several-fold higher concentrations of FGF-2 into the extracellular space of the ALSs or LTMs than the wild-type fibroblasts. These higher FGF-2 concentrations caused an increase in the rate of extracellular matrix protein synthesis by the fibroblasts within the ALSs or LTMs.

*10. Methods for Preparing and Analyzing Living Tissue Matrices (LTMs) or Autogenic Living Scaffolds (ALSs) and their Cell-Derived Matrix (CDM) component versus Fibroblast-Populated Collagen Gels and Fibrin Gels*

A. cells

Human neonatal foreskin fibroblasts (HFFs, American Type Culture Collection, Manassas, VA) were cultured in T-300 tissue culture flasks (BD Biosciences, Bedford, MA) with high glucose Dubelco's modified Eagle' medium (DMEM, Mediatech, Herndon, VA) supplemented with 10 % bovine calf serum (BCS, Hyclone, Logan, UT), and 1 % penicillin/streptomycin/ampothericin B (Invitrogen, Carlsbad, CA) at 37°C in humidified, 10 % $CO_2$ conditions. Cells were harvested at 90 % confluency with a 10 min application of 0.25 % trypsin/0.05 % EDTA solution (Mediatech). Two million, passage 5 cells were used for each sample in all experiments.

B. Standard Serum-Supplemented Medium

DMEM with 10 % fetal bovine serum (FBS, ATCC), 150 $\mu$g/ml (519 $\mu$M) L-ascorbic acid phosphate magnesium salt n-hydrate (Wako Pure Chemicals, Japan), and 1 % penicillin/streptomycin/amphotericin B (Invitrogen).

C. Chemically-Defined Medium

A 3:1 ratio of DMEM (high glucose (4.5 g/L); with L-glutamine and sodium pyruvate (Mediatech) and Ham's F12 (Invitrogen) with the addition of 5 $\mu$g/ml insulin (Sigma-Aldrich, St. Louis, MO), 5 ng/ml selenious acid (Sigma-Aldrich), $10^{-4}$ M ethanolamine (Sigma-Aldrich), 150 $\mu$g/ml L-ascorbic acid phosphate magnesium salt *n*-hydrate (Wako), 2.5 ng/ml epidermal growth factor (EGF (BD Biosciences)) in 5 $\mu$g/ml human serum albumin, EMD (Biosciences, San Diego, CA), 5 ng/ml basic fibroblast growth factor (bFGF (BD Biosciences)), $1.0 \times 10^{-7}$ M dexamethasone (Sigma-Aldrich), $2 \times 10^{-10}$ M L-3,3',5-triiodothyronine (Sigma-Aldrich), $4 \times 10^{-3}$ M Glutamax™ (Invitrogen), 1 $\mu$g/ml glutathione (reduced) (Sigma-Aldrich), and 1 % penicillin/streptomycin/amphotericin B (Invitrogen). Growth factors were added fresh at each feeding, except for **CDM, where the growth factors were added at the correct concentration (2.5 ng/ml EGF and 5.0 ng/ml bFGF) into the entire stock medium at the start of the experiment.

D. Collagen Gel, Fibrin Gel and ALS/LTM Preparation

Fibroblast-populated collagen gels (CGs) were prepared according to methods known in the art by mixing 0.2 ml of collagen stock solution (5 mg/ml of 5 mM HCl-extracted rat tail tendon collagen in 5 mM acetic acid), 0.05 ml 5X DMEM (Mediatech), 0.65 ml DMEM (Mediatech) with cells, 0.1 ml fetal bovine serum (FBS, ATCC), 150 $\mu$g L -ascorbic acid phosphate magnesium salt n-hydrate (Wako) and 1 % penicillin/streptomycin/amphotericin B (Invitrogen) at room temperature. One milliliter of the resulting solution was added into each 24 mm diameter well. The initial collagen concentration was 1.0 mg/ml, and the initial cell concentration was 2,000,000 cells/ml in 10 % FBS.

Fibroblast-populated fibrin gels (FGs) were prepared based on the methods known in the art. Briefly, HFFs in standard serum-supplemented medium were added to a fibrinogen solution (Sigma-Aldrich F4753 type IV). One-ml samples were mixed with 4 units of bovine thrombin (Sigma-Aldrich, T7513) at room temperature. One milliliter of the resulting solution was added into each 24 mm diameter well. The initial fibrinogen concentration was 1.0 mg/ml, and the initial cell concentration was 2,000,000 cells/ml in 10% FBS.

ALSs/LTMs were prepared by mixing the 2 million, passage 5 HFFs with standard serum-supplemented medium or the chemically-defined medium described above (for *CG, *CDM and **CDM) at room temperature into a final volume of 1 ml per sample. The initial cell concentration was 2,000,000 cells/ml in 10 % FBS. The 1 ml samples of collagen gels, fibrin gels, and CDMs were pipetted onto 24mm diameter, porous inserts (0.4 $\mu$m TransWells™, Corning Life Sciences, Acton, MA) suspended above standard 6-well plates, and allowed to sit undisturbed at room temperature. After a 1-hour period, 3 ml of standard serum-supplemented medium or chemically-defined medium (for *CG, *CDM and **CDM) was carefully added below, and 1 ml added above, each sample and the samples were incubated at 37 °C in humidified, 10 % $CO_2$ conditions. Samples were fed every other day (3 ml below and 2 ml above each porous insert) with the same medium for 3 weeks.

E. Mechanical Testing

After 3 weeks in culture, the samples were exposed to $ddH_2O$ for 1 hour to lyse the cells and eliminate the contractile forces produced by the fibroblasts, and then equilibrated in phosphate buffered saline (PBS, Mediatech) for biomechanical testing. The human penile skin was obtained through Analytical Biological Serives Inc. (Wilmington, DE) and shipped cold in RPMI medium with antibiotics and tested within 12 h of removal from the subject. The thickness, failure tension, failure strain, and ultimate tensile strength (UTS) of the samples were determined by a novel tissue inflation device that measures the displacement and pressure at which a sample bursts when inflated with PBS at a constant rate of 1 ml/min. The sample is circularly clamped at and inflated through a 1-cm diameter opening, thus causing the sample to form a spherical cap before failing. The increasing pressure applied to the sample was measured by an on-board pressure transducer (model PX102-025GV, Omega Engineering, Stamford, CT). The displacement of the center of the cap was measured with a laser displacement system (LDS-080, Keyence, Woodcliff Lake, NJ). The LSD-080 also measured the thickness of each sample after being slightly compressed by a small reflective disk (1.3 g, 1.3 cm diameter) for 1 minute. The maximum membrane tension, T, was calculated using the Law of Laplace for a spherical membrane:

$$T = \tfrac{1}{2}\,PR, \qquad\qquad\qquad \text{(eq. 1)}$$

where P is the pressure when the tissue bursts and R is the corresponding radius at the point of rupture, calculated assuming a spherical cap geometry by:

$$R = (w^2 + a^2)\,/\,2w, \qquad\qquad\qquad \text{(eq. 2)}$$

where a is the radius of the clamp (5 mm) and w is the displacement at the center of the sample at failure measured by the laser.

The ultimate tensile strength (UTS) was calculated by:

$$UTS = T\,/\,t, \qquad\qquad\qquad \text{(eq. 3)}$$

where t is the initial thickness of the specimen before inflation. The actual thickness at the time of bursting was less than this value. Thus the calculated UTS (engineering stress) is less than the true stress at failure, since the thickness of the specimens decreased as they were being inflated.

The ultimate tensile strength per collagen density (UTS / Collagen Density) was calculated by:

$$UTS / Collagen\ Density = UTS / (Total\ Collagen / \pi t(D/2)^2)$$
$$= T / (Total\ Collagen / \pi(D/2)^2), \qquad (eq.\ 4)$$

where D is the diameter of the constructs (2.4 cm).

The failure strain was determined from the equibiaxial strain at the pole estimated from the displacement data using the approximate relationship:

$$Failure\ Strain = 2/3\ (w/a)^2 - 2/15\ (w/a)^4 + 2/35\ (w/a)^6. \qquad (eq.\ 5)$$

F. Biochemical Analysis

Following biomechanical testing, the samples were weighed (wet weight), lyophilized overnight, and then weighed again (dry weight). Each lyophilized sample was solubilized in 1 ml of 0.5 M acetic acid and 1 mg/ml pepsin (Sigma-Aldrich) and incubated overnight at 20°C with rotation. This extraction step was repeated twice to achieve complete extraction of the acid and pepsin soluble fraction of collagen. The samples were then centrifuged at 14,000 rpm for 1 hour at 15 °C, and the supernatant was combined with samples from the other two extractions and used for determining non-acid and pepsin extractable collagen content using the Sircol™ Assay (Biocolor, Belfast, N. Ireland). The Sircol™ Assay quantified the content of intact collagen monomers in the solution, and did not detect degraded collagen (these amounts were 5-10 % of the actual collagen content (data not shown) determined by a hydroxyproline assay (see methods below)). Total non-collagenous protein content of each extract was determined with the Tp-Blue™ Total Protein Assay (Biocolor) using Coomassie brilliant blue G. Total protein content was obtained by adding this value to the total amount of collagen obtained for each sample. The remaining pellets of each sample were digested with Proteinase K (Invitrogen), 50 $\mu$g in 500 $\mu$l solution of 10 mM EDTA and 0.1 M sodium phosphate (pH 6.5) (Fisher) overnight at 60 °C. A 100 $\mu$l aliquot of the digest was used for determining sulfated glycosaminoglycan and proteoglycan content (that did not include hyaluronic acid) with the Blycan™ Assay (Biocolor). A 10 $\mu$l aliquot of the digest was then used to determine DNA content, and thus cell number (assuming 8 pg of DNA per cell), with Hoechst 33258 dye (Amersham Biosciences, Piscataway, NJ) on a DyNA Quant 200 fluorometer (Amersham Biosciences). 100-200 $\mu$l aliquots of the Proteinase K digests were used to determine the non-acid and pepsin extractable collagen content (insoluble collagen fraction) with a hydroxyproline assay. The hydroxyproline assay was based on the methods of Edwards and O'Brien (1980) and consisted of hydrolyzing each sample in 6.0 M HCl for 16 hours at 110 °C, followed by drying of the samples under vacuum, reconstituting to 2.0 ml with assay buffer (consisting of 5 g/l citric acid (Sigma-Aldrich), 1.2 ml/l glacial acetic acid (EMD Chemicals, Gibbstown, NJ), 12 g/l sodium acetate (VWR, Bridgeport, NJ), and 3.4 g/l sodium hydroxide (VWR)), mixing with 1.0 ml of Chloramine-T reagent (made from 62 mM chloramine-T solution (VWR) in 20.7 % ddH$_2$O, 26% n-propanol (VWR) and 53.3 % of assay buffer) for 20 minutes at room temperature, adding 1.0 ml of freshly prepared dimethylaminobenzaldehyde reagent (made from 15 g of *p*-dimethylaminobenzaldehyde (Sigma-Aldrich) in 60ml of n-propanol (VWR) and 26 ml of 60 % perchloric acid (VWR)) and incubating each sample at 60 °C for 15 minutes, cooling each sample in tap water for 5 minutes, and measuring the absorbance of each sample at 550 nm within 45 minutes. Absorbance readings were correlated with collagen amount using a standard curve and a conversion factor of 10 $\mu$g collagen to 1 $\mu$g 4-hydroxyproline. The standard curve was created and the conversion factor determined with known amounts of Trans-4-hydroxy-L-proline (Sigma-Aldrich) and rat tail type I collagen.

G. Histology and Transmission Electron Microscopy

One sample from each group was prepared for histological evaluation by fixing in 10 % zinc formalin (VWR) for 1 hour, followed by washing and storing in 4 °C ethanol (VWR). The samples were embedded onto paraffin blocks, sectioned into 10 $\mu$m thick sections, and stained with hematoxylin and eosin (H&E). The stained sections were imaged and photographed at 200x with a Nikon Eclipse E600 microscope and Spot digital camera (Diagnostic Instruments, Sterling Heights, MI). One sample from each group (except **CDM and penile dermis) was fixed for transmission electron microscopy (tEM) according to the methods of Gibson and Lang (1979). Briefly, the samples were fixed in 2 % glutaraldehyde for 1 hour, rinsed 3 times in sodium cacodylate buffer, fixed in osmium tetroxide for 10 minutes, and dehydrated in 10 minute ethanol (VWR) steps followed by two washes in propylene oxide (all from Electron Microscopy Sciences, Hatfield, PA). The samples were then embedded in epon-araldite resin (Electron Microscopy Sciences). Briefly, the samples were embedded in a 1:1 ratio of propylene oxide to Epon Araldite for 2 hours, followed by embedding in 100 % Epon Araldite for 3 hours, and then transferring each sample to embedding molds with fresh 100 % Epon Araldite and cured in a 60 °C oven overnight. 60-90 nm gold- and silver-colored sections were stained for 5 minutes with urinyl acetate saturated in 50 % ethanol, followed by staining for 20 minutes in lead citrate according to the methods of Venable and Coggesmall (1965). The stained sections from each of the samples were then imaged with a ZEISS electron microscope for observation of cell, collagen, and ECM morphology. The average collagen fibril diameter and density were calculated from the average measured diameters and number of collagen fibrils per square micrometer from 5 randomly chosen fields taken at high magnification (57,000X or 88,000X). The collagen diameters were measured at the thinnest point of cross-sectioned collagen fibrils with a 5x magnified ruler. If a randomly chosen field fell on a grid, then another field continued to be randomly chosen until it fell solely on the sample.

### H. Statistics

Error bars indicate one standard deviation in all figures. Statistical differences between groups were determined using ANOVA with Tukey HSD post hoc analysis (SPSS, Inc., Chicago, IL). Differences were considered significant with $p < 0.05$. Linear regression was performed and the $R^2$-values were obtained for determining the degree of association between the independent and dependent variables (SPSS, Inc.).

### Results and Discussion

Experiments were designed and carried out to determine whether the extracellular matrix (ECM) produced and assembled solely by hyperconfluent cells is mechanically superior to ECM generated by allowing cells to compact and remodel gels cast from purified solutions of collagen or fibrin. To reduce experimental variability, all groups were grown in parallel with two million human foreskin fibroblasts from the same batch, fed with the same standard serum-supplemented media at the same time, and grown for 3-weeks next to each other on the same 6-well plates. The collagen gels (CGs) and fibrin gels (FGs) started out at a thickness of 2.2 mm and contracted to a thickness 83 $\pm$ 5 $\mu$m and 218 $\pm$ 5 $\mu$m, respectively. The cell-derived matrices (CDMs), on the other hand, started out at slightly more than a single cell layer thick and grew to 125 $\pm$ 6 $\mu$m, with the synthesized ECM organizing into several alternating layers that were at approximate right-angles to each other, resembling native soft connective tissue (Fig. 17). After 3 weeks in culture, the CDMs were considerably stronger than the CGs and significantly stronger than the FGs (see Table 1).
Other measurements of CDM thickness was determined using digital image analysis, as indicated in Figure 15. Figures 15A-E show Hematoxylin and Eosin (H&E) stained sections of fibroblast-populated collagen gel (CG), fibrin gel (FG), and three cell-derived matrices (CDM, *CDM, and **CDM), respectively. All samples were grown for 3 weeks starting with the same initial number of fibroblasts. Fibroblasts were embedded in 1 mg/ml of collagen in the collagen gel, and in 1 mg/ml fibrin in the fibrin gel. The collagen and fibrin gels started at 1 mm thickness and contracted to 84 $\mu$m thick and 230 $\mu$m thick, respectively, over the first few days. The cell-derived matrices started at 2-3 cell layers thick and grew to 110 $\mu$m (CDM), 465 $\mu$m (*CDM), and 240 $\mu$m (**CDM) and in thickness, respectively, over the 3-week culture period. The CDMs were at least 5 times stronger (120 N/m for LTM versus 25 N/m for CG and FG) than the collagen and fibrin matrices (see Figure 16B) and contained numerous extracellular matrix proteins in amounts, ratios and arrangement that resembled young, native, blastema-like tissue. All micrographs were taken at 200x magnification. The thickness was measured by digital image analysis. Scale bars = 100 $\mu$m. The values for the CGs and FGs are in line with results obtained by other researchers and indicate that cell-produced ECM (i.e. CDM) can be made stronger than reconstituted ECM within a relatively short period of time.
The specification also describes a relatively simple chemically-defined medium for promoting high ECM synthesis by multilayered fibroblasts. Embodiments of the resulting minimal serum-free medium are similar to a serum-supplemented medium, except that in place of supplementation with serum, the chemically-defined medium is supplemented with basic fibroblast growth factor (bFGF), epidermal growth factor (EGF), dexamethasone and L-3,3',5-triiodothyronine, and a few other components that were determined to be necessary for cell growth that are present in serum (insulin, selenious acid, and a lipid precursor). The presence of these few components instead of serum resulted in a doubling (**CDMs: growth factors added into the stock medium at the start of the 3-week culture period) or tripling (*CDMs: growth factors added fresh at each feeding) in thickness (see Table 1, and Figure 16A), and a 40% (*CDM or 210% (**CDM) increase in the ultimate tensile strength (UTS) (Table 1, and Figure 16F) over standard serum-supplemented samples (CDM). The matrix produced using either version of the chemically-defined medium also resulted in a doubling of the failure strain (Table 1 and Figure 16J), a quadrupling of the proliferation rate (Table 1 and Figures 16I), and a quintupling of the fraction of non-acid and pepsin extractable collagen (Table 1 and Figure 16K) - a parameter that is highly correlated (adjusted $R^2 = 0.977$) with the strength of the sample matrices. Interestingly, when collagen gels were fed with the chemically-defined medium (*CG), the contractile forces within the collagen gels became so great that they detached from the wells and contracted into themselves within 24 h of feeding them with the chemically-defined medium. This excessive contraction prevented a comparison of the effects of the chemically-defined medium and the serum-supplemented medium on the development of gels and is the subject of further studies.

In addition, the structural characteristics and biochemical composition between the five types of matrix were compared, as well as with native penile skin, to determine how these differences correlated with their biomechanical properties. These studies were done to provide further insight into the mechanisms of cell-mediated strengthening of ECM. Human penile skin was to compare native soft connective tissue to the 5 different living tissue equivalents (LTEs) produced in this study, even though it is several-fold weaker than normal skin, since the fibroblasts used in making the LTEs were derived from neonatal human foreskin.

Although it is known that fibroblasts entrapped in purified collagen synthesize far less ECM proteins than in monolayer cultures, while fibroblasts entrapped in purified fibrin retain their ability to produce ECM protein, our data also surprisingly demonstrate that fibroblasts in self-produced collagen-rich matrices produce an even greater amount of ECM proteins such as collagen than in purified fibrin gels. Moreover, although the cell-derived matrices synthesized a similar amount of collagen as that of the collagen gels, the CDM-synthesized collagen did not appear to result in any inhibition of ECM synthesis in the cell-derived matrices. Specifically, the total protein synthesized by the cells in the cell-derived matrices was significantly more than the net increase in total protein in the collagen gels and fibrin gels (see Table 1 and Figure 16E).

These values are higher but in line with the results obtained by other researchers for gels and cell-produced matrices (Grinnell *et al.*,1989; Huang et al., 1993; Clark et al., 1997; Neidert *et al.,* 2002). These differences could be due to the much greater cell density of our cultures that might have signaled the fibroblasts to synthesize more ECM as in the CDM cultures. These differences might also be due to the use of porous inserts that provided a shorter diffusion distance for nutrients for cells on the basal side of the samples (the use of porous inserts resulted in a more than 50 % increase in thickness (but no effect on UTS) over samples grown on regular 6-well plates (data not shown)), or the 80% greater concentration of L-ascorbate, in the more stable form of L-ascorbic acid phosphate magnesium salt *n*-hydrate (1 week stability versus 24 h stability for L-ascorbate).

The cell-derived matrices also had a significantly greater fraction of non-acid and pepsin extractable collagen (insoluble collagen fraction) than the collagen and fibrin gels (see Table 1 and Figure 16K). The non-acid and pepsin extractable collagen fraction was the amount of collagen that was not solubilized by repeated 0.5 M acetic acid and pepsin (1 mg/ml) extraction steps over a 3-day period. The non-acid and pepsin extractable collagen density provides a measure of very stable collagen in the tissues that is highly cross-linked or bundled to resist extraction. The UTS / Collagen Density, a metric that is independent of the thickness of the samples and represents the strength of the constructs normalized per unit of collagen, was likewise significantly greater for the cell-derived matrices than the collagen gels (Table 1 and Figure 16L). The high value of UTS /Collagen Density for the fibrin gels ($25.9 \pm 2.4$ kPa/mg/cm$^3$- see Table 1) might be due to ECM proteins other than collagen, such as fibrin, in the gel. However, others have found that the UTS of fibrin gels are correlated to the total collagen amount in the constructs ($R^2 = 0.77$ at day 21), and our study found that the UTS correlated to the collagen density ($R^2 = 0.73$ at day 21) not just in fibrin gels but also in collagen gels and cell-derived matrices. Our results indicate that UTS of the CDMs of the presently claimed invention correlate even more closely to the non-acid and pepsin extractable collagen density ($R^2 = 0.993$) than to collagen density. Thus, the strength of living tissue equivalents as described here, as well as native soft connective tissue, is not just correlated with collagen density, but more specifically to a very stable form of collagen represented by the non-acid and pepsin extractable collagen density.

The strength of soft connective tissues, as well as living tissue equivalents such as fibroblast-populated collagen gels, is generally attributed to the collagen density and average collagen fibril diameter. As summarized in Table 1, observation by transmission electron microscopy (tEM) revealed that the diameter of the collagen fibrils in the CDMs, *CDMs, collagen gels and fibrin gels were $46 \pm 5$ nm, $48 \pm 5$ nm, $52 \pm 10$ nm and $47 \pm 5$ nm, respectively, while the collagen density was significantly greater for the CDMs ($79 \pm 2$ fibrils/$\mu$m$^2$), *CDMs ($80 \pm 2$ fibrils/$\mu$m$^2$) and collagen gels ($81 \pm 4$ fibrils/$\mu$m$^2$) than for the fibrin gels ($28 \pm 4$ fibrils/$\mu$m$^2$, p < 0.01). These diameters fall within the range of collagen fibril diameters (40 -100 nm) found in native soft connective tissue.

In the present case, although most of the collagen in the collagen gel consisted of large, approximately 56 nm diameter fibrils, there was a small presence of approximately 46 nm diameter fibrils; presumably newly synthesized collagen (see Fig. 17), indicating that some of the original collagen in the gel had been degraded since the total amount of collagen in the collagen gels remained unchanged over the 3-week period. And although the collagen gels disclosed herein had by far the greatest collagen density and the largest collagen fibril diameters, they were significantly weaker than the cell-derived matrices grown under identical conditions. As can be seen in Figures 17A-17D, distribution of collagen fibrils. CDMs, collagen gels, and *CDMs had a more than 2.5 times greater density of collagen fibrils at around 80 fibrils/$\mu$m$^2$ than fibrin gels at 28 fibrils/$\mu$m$^2$. Collagen fibrils are shown by arrows, and all micrographs were taken at 12,000x with the scale bar = 1 $\mu$m. The collagen fibrils of the cell-derived matrices (Figures 17C and D) appeared more parallel and to consist of more parallel alternating layers than any of the other groups (see upper portion of CDM where the micrograph consists of cross-sections of thousands of parallel collagen fibrils). The collagen fibrils of the collagen gels (Figures 17A) appeared somewhat parallel but consisted of only a few alternating layers, while the collagen fibrils of the fibrin gels (Figure 17B) appeared only parallel in small regions of the ECM.

Without being limited to theory, this discrepancy could be due to a greater presence of intra-molecular covalent cross-links between the collagen fibrils or more extensive bundling of the collagen fibrils into fibers in the cell-derived matrices than in the collagen gels and fibrin gels. The non-acid and pepsin extractable collagen fraction was closely correlated to the UTS / Collagen Density values for all these groups ($R^2$ = 0.93), except for the fibrin gels ($R^2$ = 0.11). The *CDMs and **CDMs, which had a several-fold greater fraction of non-acid and pepsin extractable collagen than any of the other samples, also had a higher thermal and enzymatic stability than any of the other samples (data not shown). The very stable collagen represented by the non-acid and pepsin extractable collagen fraction appears to consist of highly cross-linked or bundled collagen that resists extraction and gives rise to a more resistant structure that leads to strengthening of the matrix.

Looking at Figure 16, a graphical depiction of the data in Table 1, it can be seen in Figures 16B and 16F that all types of cell-derived matrices were stronger than collagen gels (CG) and fibrin gels (FG). *CDMs and **CDMs were significantly thicker (Figure 16A) and contained far more total protein (Figure 16E) than their serum-supplemented companions (CDMs, collagen gels and fibrin gels), but had only half the percentage of proteoglycans and glycosaminoglycans (Figures 16D and 16H). *CDMs and **CDMs also contained a significantly higher fraction of non-acid and pepsin extractable collagen (Figure 16K), and were stronger per microgram of collagen (UTS/Collagen Density) than collagen gels or fibrin gels (Figure 16L). CDMs were more than twice as strong as collagen gels per microgram of collagen, indicating the possibility that the collagen in the CDMs was more organized for strength than in collagen gels. In addition to collagen, a substantial proportion of the strength of *CDMs and fibrin gels was also due to other ECM proteins. *CDMs and **CDMs were significantly more extensible than CDMs, collagen gels and fibrin gels. Human penile skin (HPS) most closely resembled **CDM mechanically, and *CDM and **CDM biochemically.

The lowered growth factor concentration over time that occurs naturally *in vivo* during the wound healing response, where the high concentration of growth factors in a fresh clot decreases over time as it is invaded and reconstructed by fibroblasts, was approximated by adding the growth factors (EGF and bFGF) directly into the feeding media at the start of the 3-week culture period and taking advantage of the natural loss of growth factor activity in media over time. This one-time addition of growth factors at the start of the 3-week period (**CDM) resulted in a matrix with about ½ the thickness of parallel samples (*CDM) for which the growth factors were added fresh at every feeding (see Table 1 and Figure 16A). The thickness was highly correlated with total protein content ($R_{adj}^2$= 0.9), which in turn correlated with total cell number ($R_{adj}^2$= 0.874. Interestingly, the **CDMs had significantly more total collagen than *CDMs, although the *CDMs had almost twice as much total protein as the **CDMs (Table 1 and Figure 16C). Since collagen is the major extracellular matrix protein providing strength, the **CDMs, with a more than two-fold greater fraction of collagen than the *CDMs, were also almost twice as strong as the *CDMs (Table 1 and Figure 16F).

Cell-derived matrices grown for longer periods (up to 6 weeks) further continued to increase in thickness and strength (data not shown). It is possible that the **CDMs developed mostly in thickness for the first part of the growth period followed by development in strength for the remainder of the growth period, since adding lower concentrations of fresh growth factors at every feeding instead resulted in matrices that were significantly stronger than the *CDMs but significantly thinner than the **CDMs (data not shown). This effect appears to be due to the fact that EGF stimulates the synthesis of non-collagenous proteins but inhibits the transcription of type 1 collagen genes, while high bFGF concentrations favor increased cell proliferation over enhancing the synthesis and strengthening of the ECM. Thus a decrease in total growth factor activity over time results in less synthesis of non-collagenous proteins, but an increase in the strength of the synthesized ECM by the resulting increase in collagen synthesis. Many growth factors, including bFGF, can also accumulate and retain their activity for relatively long periods of time within ECM. Thus some of the added growth factors might have accumulated in the ECM during the first part of the culture period and then used up continuously as the matrix developed. The failure strains for the *CDMs and **CDMs were also significantly greater than for their serum-supplemented counterparts (Table 1 and Figure 16J), closer mimicking native soft connective tissue.

These results demonstrate that living tissue equivalents are stronger and thicker when they are cell-produced - in other words, when the cells are allowed to grow and develop their own mechanical environment rather than being supplied with a scaffold, such as gels or other biopolymer materials. This strength appears to be due to a greater fraction of very stable collagen and the significantly greater synthetic rate of fibroblasts in ALSs/LTMs than in gels. The LTMs and their cell-derived matrix component (*CDM and **CDM) produced and described herein have potential for use as soft connective tissue substitutes since they are produced solely from cells fed with a chemically-defined medium that does not contain animal components. The rapid growth and lack of expensive serum makes the development of LTMs as soft connective tissue substitutes commercially more viable, and opens the door to mainstream acceptance and appeal.

Due to the relative simplicity of the chemically-defined medium, these cell-produced living tissue equivalents may also serve as *in vitro* biological models for the effects of nutritional components and pharmaceutical products on the growth and development of soft connective tissue, for examining the numerous *in vivo* conditions and processes such as wound healing and connective tissue formation, and for investigating the development and interaction of different cells and tissues in a soft connective tissue environment that is developed solely from cells *in vitro.* The higher thermal and enzymatic stability and mechanical integrity of cell-derived matrices over both collagen and fibrin gels may also allow them to retain their structural integrity longer in *in vivo* conditions. The ability to grow LTMs thick and strong in a relatively short period of time in chemically-defined conditions provides a commercially viable option for wound repair and tissue regeneration as an attractive alternative to the use of fibrin gels, collagen gels and even native tissues.

**Table 1**. Results from mechanical and biochemical analysis of human penile skin and cell-derived matrices (CDMs, *CDMs, **CDMs), fibroblast-populated collagen gels and fibrin gels containing human foreskin fibroblasts. Numbers indicate mean +/- SD. Numbers in parentheses indicate approximate initial amount. Collagen fibril diameters and densities were measured in proximity to cell surfaces.

| Measure | Collagen gel | Fibrin gel | CDM | *CDM | **CDM | Penile Skin |
|---|---|---|---|---|---|---|
| Ultimate Tensile Strength (kPa) | 168.5 ± 43.1 | 133.2 ± 10.6 | 223.2 ± 9 | 314.5 ± 7.2 | 697.1 ± 36.1 | 713.0 ± 55.2 |
| Thickness ($\mu$m) | 83 ± 5 (2,220 $\mu$m) | 218 * 5 (2,220 $\mu$m) | 125 ± 6 (-30 $\mu$m) | 395 ± 6 (-30 $\mu$m) | 225 ± 7 (-30 $\mu$m) | 651 ± 30 |
| Failure Strain | 0.13 ± 0.02 | 0.21 ± 0.03 | 0.18 ± 0.03 | 0.33 ± 0.03 | 0.31 ± 0.06 | 0.88 ± 0.28 |
| Total Protein (mg) | 1.08 ± 0.03 (1.0 mg) | 1.58 ± 0.04 (1.0 mg) | 1.25 ± 0.06 | 4.40 ± 0.08 | 2.65 ± 0.07 | 6.74 ± 0.44 |
| Total Collagen (mg) | 0.99 ± 0.02 (1.0 mg) | 0.51 ± 0.03 | 0.85 ± 0.03 | 1.40 ± 0.04 | 1.78 ± 0.06 | 3.98 ± 0.37 |
| Non-Acid and Pepsin Extractable Collagen Fraction (%) | 1.5 ± 0.1 | 1.3 ± 0.1 | 2.3 ± 0.2 | 12.8 ± 0.5 | 13.1 ± 0.3 | 15.7 ± 0.7 |
| Collagen Density (mg/cm$^3$) | 26.5 ± 1.5 | 5.2 ± b.2 | 15.1 ± 0.9 | 7.8 ± 0.2 | 17.4 ± 0.1 | 13.5 ± 0.7 |
| UTS / Collagen Density (kPa/mg/cm$^3$) | 6.4 ± 1.9 | 25.9 ± 2.4 | 14.5 ± 1.1 | 40.3 ± 0.4 | 40.0 ± 1.9 | 52.9 ± 3.1 |
| Collagen Fibril Diameter (nm) | 52 ± 10 | 47 ± 5 | 46 ± 5 | 48 ± 5 | - | - |
| Collagen Fibril Density (fibrils/$\mu$m$^2$) | 81 ± 4 | 28 ± 4 | 79 ± 2 | 80 ± 2 | - | - |
| Total Proteoglycans & Glycosaminoglycans ($\mu$g) | 38.4 ± 0.7 | 45.4 ± 0.9 | 31.3 ± 0.5 | 64.3 ± 0.6 | 38.4 ± 0.4 | 83.0 ± 5.7 |
| Wet Weight / Dry Weight | 16.4 ± 0.6 | 20.1 ± 0.4 | 13.0 ± 0.5 | 19.5 ± 0.2 | 20.3 ± 0.6 | 21.7 ± 0.4 |
| Cell Number (million) | 2.8 ± 0.1 (2.0 million) | 4.0 ± 0.1 (2.0 million) | 2.8 ± 0.1 (2.0 million) | 6.1 ± 0.1 (2.0 million) | 3.6 ± 0.1 (2.0 million) | 9.8 ± 0.4 |

**Claims**

1. A living three-dimensional construct comprising fibroblast cells and an extracellular matrix synthesized *in vitro* by said fibroblast cells, wherein said construct is obtainable by a process comprising:

   incubating the fibroblast cells for 3 weeks in the following chemically-defined medium:

(a) 3:1 ratio of DMEM high glucose (4.5 g/L); with L-glutamine and sodium pyruvate and Ham's F12 medium with the addition of;
(b) 5 $\mu$g/ml insulin;
(c) 5 ng/ml selenious acid;
(d) $10^{-4}$ M ethanolamine;
(e) 150 $\mu$g/ml L-ascorbic acid phosphate magnesium salt n-hydrate;
(f) 2.5 ng/ml epidermal growth factor in 5 $\mu$g/ml human serum albumin;
(g) 5 ng/ml basic fibroblast growth factor;
(h) 1.0 x $10^{-7}$ M dextramethasone;
(i) 2 x $10^{-10}$ M L-3.3',5-triiodothyronine;
(j) 4 x $10^{-3}$ M Glutamax™;
(k) 1 $\mu$g/ml glutathione; and
(l) 1% penicillin/streptomycin/amphotericin B.

2. The construct of claim 1 having a failure strain of at least 0.2.

3. The construct of claim 1 or claim 2 wherein the thickness is at least 100 $\mu$m.

4. The construct of any one of claims 1 to 3, wherein the construct further comprises additional cells, tissue or any combination thereof.

5. The construct of claim 4, wherein said additional cells or tissue are of a type other than said fibroblast cells.

6. The construct of any one of claims 1 to 5 that promotes any of cell growth, proliferation, migration and/or acquisition of *in vivo-like* morphology or any combination thereof.

7. The construct of claim 6, wherein said cell growth, proliferation, migration and/or acquisition of *in vivo*-like morphology or any combination thereof may occur *in vivo* or *in vitro.*

8. The construct of claim 4, wherein said additional cells, tissue or any combination thereof may include stem cells, progenitor cells, precursor cells; cells or tissue of a connective, epithelial, muscle, nerve or glandular origin; and cells of vascular and/or non-vascular organ origin including neuroblastomas, myoblasts, astrocytes, cardiomyocytes, skeletal muscle myoblasts, hepatocytes, chondrocytes, osteoblasts, fibroblasts, keratinocytes, Schwann cells, nerve cells, glial cells, epithelial cells, endothelial cells, smooth muscle cells, skeletal muscle cells, cardiac muscle cells, stromal cells, mesangial cells, mesenchymal cells, hematopoietic cells, dendritic cells, immune system cells, neural tissue, hepatic tissue, aortic tissue, venous tissue, capillary tissue, cartilage, bone, muscle, glands, or hair follicles.

9. The construct of claim 8, wherein said construct supports the growth of cells, tissue, organs, or any combination thereof.

10. The construct of claim 4, wherein said construct provides nutritional support, developmental support, or a combination thereof.

11. The construct of claim 4, wherein said construct provides mechanical and structural support.

12. The construct of claim 4, wherein said construct comprises neuronal cells.

13. A method for using the construct as defined in any one of claims 1 to 12, comprising modeling a human biological cellular system or tissue system or a combination thereof *in vitro.*

14. The method of claim 13, wherein said cells are fed with serum-free media.

15. The method of claim 14, wherein said serum-free media comprises:

(a) base media
(b) basic fibroblast growth factor (bFGF) or equivalent growth factor;
(c) insulin;
(d) glucocorticoid; and

(e) selenium;

at concentrations suitable to sustain fibroblast growth.

16. The method of claim 15, wherein said serum-free media further comprises one or more of:

(a) extra L-glutamine (preferably in the form of Glutamax™);
(b) an additional growth factor (epidermal growth factor (EGF), platelet derived growth factor (PDGF), etc.)
(c) L-3,3',5-triiodothyronine;
(d) a lipid precursor including ethanolamine or equivalent, i.e. o-phosphorylethanolamine; ascorbic acid (preferably in the stable form of L-ascorbic acid phosphate magnesium salt n-hydrate);
(e) ascorbic acid;
(f) an antioxidant (such as glutathione (reduced) or Trolox™);
(g) extra specific amino acid(s);
(h) any other nutrient or factor that is compatible with cell growth; and
(i) any other nutrient or factor that is compatible with production of extracellular matrix.

17. The method of claim 16, wherein said serum-free media further comprises any other nutrient or factor that is compatible with cell growth and secretion of growth factors and/or other compounds that promote the growth and development of the seeded cells or tissue of interest.

18. The method of claim 17, wherein said media comprises:

(a) 3:1 ratio of DMEM (high glucose with L-glutamine and sodium pyruvate):F12 or 2:1 ratio of IMEM:F12;
(b) 4 mM Glutamax™;
(c) 2.5 ng/ml EGF in human serum albumin;
(d) 5.0 ng/ml bFGF
(e) 90 $\mu$M insulin;
(f) 100 nM Dexamethasone;
(g) 200 pM L-3,3',5-triiodothyronine;
(h) 100 $\mu$M ethanolamine;
(i) 40 nM selenious acid;
(j) 320 $\mu$M L-ascorbic acid phosphate magnesium salt n-hydrate;
(k) 1 $\mu$g/ml reduced glutathione; and
(l) 1% pen/strep/ampB.

19. The method of any one of claims 13 to 18, wherein the construct is seeded with additional cells, tissue or combination thereof.

20. The method of claim 19, wherein said additional cells, tissue or a combination thereof comprise stem cells, progenitor cells, precursor cells; cells or tissue of a connective, epithelial, muscle, nerve and/or glandular origin; and cells of vascular and/or non-vascular organ origin such as neuroblastomas, myoblasts, astrocytes, cardiomyocytes, skeletal muscle myoblasts, hepatocytes, chondrocytes, osteoblasts, fibroblasts, keratinocytes, Schwann cells, nerve cells, glial cells, epithelial cells, endothelial cells, smooth muscle cells, skeletal muscle cells, cardiac-muscle cells, stromal cells, mesangial cells, mesenchymal cells, hematopoietic cells, dendritic cells, immune system cells, neural tissue, hepatic tissue, aortic tissue, venous tissue, capillary tissue, cartilage, bone, muscle, glands, or hair follicles.

21. The method of claim 19 or 20, wherein said additional cells, tissue or combination thereof comprise more than one type of additional cells.

22. The method of any one of claims 19 to 21, wherein said additional cells, tissue or combination thereof comprise neuroblastomas and myoblasts.

23. The method of any one of claims 19 to 22, wherein said additional cells or are from a particular individual and said biological system being modeled is unique to said particular individual.

24. The method of any one of claims 19 to 23, wherein said additional cells, tissue or combination thereof are from a particular biological source and said biological system being modeled is unique to said particular biological source.

**25.** The method of claim 24, wherein said particular biological source comprises a source of connective, epithelial, muscle, nerve, circulatory including hormonal, immune, vascular or non-vascular organ origin.

**26.** The method of claim 24 or 25, wherein said particular biological source further includes a microbial agent selected from the group consisting of viral, prion, bacterial, fungal, and parasitic agents.

**27.** The method of claim 26, wherein said particular biological source is hepatocyte cells or liver tissue and said microbial agent is a hepatitis virus.

**28.** The method of claim 27, further comprising assessing the effect of one or more additional agents on said biological system being modeled, wherein said one or more agents comprises pharmaceutical agents, enzymes, hormones, small molecules, peptides, polypeptides, natural products, natural products extracts, inorganic salts, other cells, growth factors, clotting factors, toxins, poisons, nucleic acids, mechanical stress inducers, electrical current generators, electromagnetic field and pulse generators, and/or sonic wave inducers.

**29.** A method of using the construct as defined in any one of claims 1 to 12 to grow tissue comprising the steps:

> (a) seeding said construct with additional cells and/or tissue;
> (b) feeding with media favorable for the growth and development of said seeded cells and/or tissue;
> (c) maintaining said seeded cells within the construct for a period of time sufficient to allow tissue growth.

**30.** The method of claim 29, further comprising maintaining said seeded cells and/or tissue within said construct under conditions that promote three-dimensional tissue growth.

**31.** The method of claim 29 or 30, wherein said seeded cells and/or tissue include cells and/or tissue of connective, epithelial, muscle, nerve, or glandular tissue, or include cells and/or tissue of vascular or non-vascular organs, or any combination thereof.

**32.** A method of producing differentiated cells and/or tissue from the construct of any one of claims 1 to 12, comprising any one of:

> (a) seeding said construct with cells and/or tissue;
> (b) contacting said seeded construct with at least one growth factor such as basic fibroblast growth factor (bFGF), epidermal growth factor (EGF), brain derived nerve growth factor (BDNF);
> (c) contacting said seeded construct with differentiated cells;
> (d) contacting said seeded construct with tissue;
> (e) adding growth medium with or without serum such as fetal human serum (FHS) to said seeded construct;
> (f) placing said seeded construct in a directional electromagnetic field;
> (g) placing said seeded construct in a pre-formed mold having a particular hollow shape, such as a balloon-shaped or disc-shaped mold;
> (h) placing said seeded construct in a simulated near-zero-gravity culture environment;
> (i) contacting said construct with a sample of differentiated cells;
> (j) providing said seeded construct with appropriate growth conditions including mechanical conditions such as stress or strain, chemical conditions such as supplemental nutrients or growth factors, and environmental conditions such as altered temperature, pH, or atmospheric pressure to promote differentiation and tissue regeneration; or
> (k) maintaining said seeded construct in a viable condition such that tissue is regenerated.

**33.** A method for generating viable vertebrate neuronal tissue *in vitro* comprising:

> (a) seeding the construct of any one of claims 1 to 12 with vertebrate primary neural cells or neuronal tissue; and
> (b) maintaining said seeded construct in culture under conditions where said viable vertebrate neuronal tissue is generated.

**34.** The method of claim 33, wherein said construct is seeded with vertebrate neural progenitor cells, neural stem cells, or neuroblastomas, preferably vertebrate glial cells.

**35.** The method of claim 34, wherein said vertebrate neuronal tissue is mammalian nerve tissue, preferably human nerve tissue.

**Patentansprüche**

1.  Lebendes dreidimensionales Konstrukt, das Fibroblastenzellen und eine extrazelluläre Matrix, die durch diese Fibroblastenzellen in vitro synthetisiert ist, aufweist, wobei das Konstrukt mittels eines Verfahrens erhaltbar ist, das umfasst:

    Inkubieren der Fibroblastenzellen für 3 Wochen in dem folgenden chemisch definierten Medium:

    (a) 3:1-Verhältnis von DMEM Hochglukose (4,5 g/l); mit L-Glutamin und Natriumpyruvat und Ham's F12-Medium unter Zugabe von:
    (b) 5 $\mu$g/ml Insulin;
    (c) 5 ng/ml selenige Säure;
    (d) 10-4 M Ethanolamin;
    (e) 150 $\mu$g/ml L-Ascorbinsäurephosphat-Magnesiumsalz n-Hydrat;
    (f) 2,5 ng/ml epidermaler Wachstumsfaktor in 5 $\mu$g/ml humanem Serumalbumin;
    (g) 5 ng/ml basischer Fibroblasten-Wachstumsfaktor;
    (h) 1,0 x 10-7 M Dextramethason;
    (i) 2 x 10-10 M L-3,3',5-Triiodothyronin,
    (j) 4 x 10-3 M GlutamaxTM;
    (k) 1 $\mu$g/ml Glutathion; und
    (l) 1 % Penicillin/Streptomycin/Amphotericin B.

2.  Konstrukt gemäß Anspruch 1 mit einer Versagensdehnung von wenigstens 0,2.

3.  Konstrukt gemäß Anspruch 1 oder Anspruch 2, wobei die Dicke wenigstens 100 $\mu$m beträgt.

4.  Konstrukt gemäß einem der Ansprüche 1 bis 3, wobei das Konstrukt außerdem zusätzliche Zellen, Gewebe oder irgendeine Kombination davon aufweist.

5.  Konstrukt gemäß Anspruch 4, wobei die zusätzlichen Zellen oder Gewebe von einem anderen Typ als die Fibroblastenzellen sind.

6.  Konstrukt gemäß einem der Ansprüche 1 bis 5, das irgendeines von Zellwachstum, Vermehrung, Migration und/oder Erwerb einer in vivo-ähnlichen Morphologie oder irgendeine Kombination davon fördert.

7.  Konstrukt gemäß Anspruch 6, wobei das Zellwachstum, Vermehrung, Migration und/oder Erwerb einer in vivo-ähnlichen Morphologie oder irgendeine Kombination davon in vivo oder in vitro erfolgen kann.

8.  Konstrukt gemäß Anspruch 4, wobei die zusätzlichen Zellen, Gewebe oder irgendeine Kombination davon Stammzellen, Progenitorzellen, Vorläuferzellen; Zellen oder Gewebe eines Bindegewebs-, Epithel-, Muskel-, Nerven- oder Drüsen-Ursprungs; und Zellen eines vaskulären und/oder nicht-vaskulären Organ-Ursprungs, einschließlich Neuroblastome, Myoblasten, Astrozyten, Cardiomyozyten, Skelettmuskel-Myoblasten, Hepatozyten, Chondrozyten, Osteoblasten, Fibroblasten, Keratinozyten, Schwann-Zellen, Nervenzellen, Gliazellen, Epithelzellen, Endothelzellen, glatter Muskelzellen, Skelettmuskelzellen, Herzmuskelzellen, Stromazellen, mesangialen Zellen, mesenchymalen Zellen, hematopoetischen Zellen, dendritischen Zellen, Immunsystemzellen, neuralen Gewebes, hepatischen Gewebes, aortischen Gewebes, venösem Gewebe, kapillarem Gewebe, Knorpel, Knochen, Muskel, Drüsen oder Haarfollikeln, umschließen können.

9.  Konstrukt gemäß Anspruch 8, wobei das Konstrukt das Wachstum von Zellen, Gewebe, Organen oder irgendeiner Kombination davon fördert.

10. Konstrukt gemäß Anspruch 4, wobei das Konstrukt alimentäre Förderung, entwicklungsbezogene Förderung oder eine Kombination davon bietet.

11. Konstrukt gemäß Anspruch 4, wobei das Konstrukt mechanische und strukturelle Förderung bietet.

**12.** Konstrukt gemäß Anspruch 4, wobei das Konstrukt neuronale Zellen aufweist.

**13.** Verfahren zur Verwendung des Konstrukts, wie in einem der Ansprüche 1 bis 12 definiert, das die Modellierung eines humanen biologischen zellulären Systems oder Gewebesystems oder einer Kombination davon in vitro umfasst.

**14.** Verfahren gemäß Anspruch 13, wobei die Zellen mit Serum-freiem Medium gefüttert werden.

**15.** Verfahren gemäß Anspruch 14, wobei das Serum-freie Medium aufweist:

(a) Basismedium;
(b) basischer Fibroblasten-Wachstumsfaktor (bFGF) oder äquivalenter Wachstumsfaktor;
(c) Insulin;
(d) Glukocorticoid; und
(e) Selenium;

bei Konzentrationen, die zur Aufrechterhaltung des Fibroblasten-Wachstums geeignet sind.

**16.** Verfahren gemäß Anspruch 15, wobei das Serum-freie Medium außerdem ein oder mehrere aufweist von:

(a) zusätzlich L-Glutamin (vorzugsweise in der Form von GlutamaxTM);
(b) einem zusätzlichen Wachstumsfaktor (epidermaler Wachstumsfaktor (EGF), von Blutplättchen abgeleiteter Wachstumsfaktor (PDGF) etc.)
(c) L-3,3',5-Triiodothyronin;
(d) einem Lipid-Vorläufer, einschließlich Ethanolamin oder einem Äquivalent, d.h. o-Phosphorylethanolamin; Ascorbinsäure (vorzugsweise in der stabilen Form des L-Ascorbinsäurephosphat-Magnesiumsalz n-Hydrats);
(e) Ascorbinsäure;
(f) einem Antioxidationsmittel (wie etwa Glutathion (reduziert) oder TroloxTM);
(g) zusätzlicher spezifischer Aminosäure(n);
(h) irgendeinem anderen Nährstoff oder Faktor, der mit dem Zellwachstum kompatibel ist; und
(i) irgendeinem anderen Nährstoff oder Faktor, der mit der Produktion von extrazellulärer Matrix kompatibel ist.

**17.** Verfahren gemäß Anspruch 16, wobei das Serum-freie Medium außerdem irgendeinen anderen Nährstoff oder Faktor aufweist, der mit Zellwachstum und Sekretion von Wachstumsfaktoren und/oder anderen Verbindungen, die das Wachstum und die Entwicklung der ausgesäten Zellen oder Gewebe von Interesse fördern, kompatibel ist.

**18.** Verfahren gemäß Anspruch 17, wobei das Medium aufweist:

(a) 3:1-Verhältnis von DMEM (Hochglukose mit L-Glutamin und Natriumpyruvat) : F12, oder 2:1-Verhältnis von IMEM : F12;
(b) 4 mM GlutamaxTM;
(c) 2,5 ng/ml EGF in humanem Serumalbumin;
(d) 5,0 ng/ml bFGF;
(e) 90 $\mu$M Insulin;
(f) 100 nM Dexamethason;
(g) 200 pM L-3,3'-5-Triiodothyronin;
(h) 100 $\mu$M Ethanolamin;
(i) 40 nM selenige Säure;
(j) 320 $\mu$M L-Ascorbinsäurephosphat-Magnesiumsalz n-Hydrat;
(k) 1 $\mu$g/ml reduziertes Glutathion; und
(l) 1% Pen/Strep/AmpB.

**19.** Verfahren gemäß einem der Ansprüche 13 bis 18, wobei das Konstrukt mit zusätzlichen Zellen, Gewebe oder einer Kombination davon besät wird.

**20.** Verfahren gemäß Anspruch 19, wobei die zusätzlichen Zellen, Gewebe oder eine Kombination davon Stammzellen, Progenitorzellen, Vorläuferzellen; Zellen oder Gewebe eines Bindegewebs-, Epithel-, Muskel-, Nerven- und/oder Drüsen-Ursprungs; und Zellen eines vaskulären und/oder nicht-vaskulären Organ-Ursprungs, wie etwa Neuroblastome, Myoblasten, Astrozyten, Cardiomyozyten, Skelettmuskel-Myoblasten, Hepatozyten, Chondrozyten, Osteoblasten, Fibroblasten, Keratinozyten, Schwann-Zellen, Nervenzellen, Gliazellen, Epithelzellen, Ehdothelzellen, glatte Muskelzellen, Skelettmuskelzellen, Herzmuskelzellen, Stromazellen, measangiale Zellen, mesenchymale Zellen, hematopoetische Zellen, dendritische Zellen, Immunsystemzellen, neurales Gewebe, hepatisches Gewebe, aortisches Gewebe, venöses Gewebe, kapillares Gewebe, Knorpel, Knochen, Muskel, Drüsen oder Haarfollikeln, aufweisen.

21. Verfahren nach Anspruch 19 oder 20, wobei die zusätzlichen Zellen, Gewebe oder eine Kombination davon mehr als einen Typ von zusätzlichen Zellen aufweisen.

22. Verfahren gemäß einem der Ansprüche 19 bis 21, wobei die zusätzlichen Zellen, Gewebe oder eine Kombination davon Neuroblastome und Myoblasten aufweisen.

23. Verfahren gemäß einem der Ansprüche 19 bis 22, wobei die zusätzlichen Zellen von einem bestimmten Individuum sind und das zu modellierende biologische System für das bestimmte Individuum spezifisch ist.

24. Verfahren gemäß einem der Ansprüche 19 bis 23, wobei die zusätzlichen Zellen, Gewebe oder eine Kombination davon von einer bestimmten biologischen Quelle sind und das zu modellierende biologische System für die bestimmte biologische Quelle spezifisch ist.

25. Verfahren gemäß Anspruch 24, wobei die bestimmte biologische Quelle eine Bindegewebs-, Epithel-, Muskel-, Nerven-, Zirkulations-Quelle, einschließlich eines hormonellen, immunologischen, vaskulären oder nicht-vaskulären Organ-Ursprungs, aufweist.

26. Verfahren gemäß Anspruch 24 oder 25, wobei die bestimmte biologische Quelle außerdem ein mikrobielles Agens enthält, ausgewählt aus der Gruppe bestehend aus viralen, prionischen, bakteriellen, fungalen und parasitären Agentien.

27. Verfahren gemäß Anspruch 26, wobei die bestimmte biologische Quelle hepatozytische Zellen oder Lebergewebe ist und das mikrobielle Agens ein Hepatitis-Virus ist.

28. Verfahren gemäß Anspruch 27, das außerdem das Beurteilen der Wirkung der ein oder mehreren zusätzlichen Agentien auf das zu modellierende biologische System umfasst, wobei die ein oder mehreren Agentien pharmazeutische Agentien, Enzyme, Hormone, Kleinmoleküle, Peptide, Polypeptide, natürliche Produkte, Extrakte aus natürlichen Produkten, anorganische Salze, andere Zellen, Wachstumsfaktoren, Gerinnungsfaktoren, Toxine, Giftstoffe, Nukleinsäuren, mechanische Stressinduktoren, elektrische Stromgeneratoren, elektromagnetische Feld- und Pulsgeneratoren und/oder Schallwellen-Induktoren aufweisen.

29. Verfahren der Verwendung des Konstrukts, wie in einem der Ansprüche 1 bis 12 definiert, zum Züchten von Gewebe, das die folgenden Schritte umfasst:

(a) Besäen des Konstrukts mit zusätzlichen Zellen und/oder Gewebe;
(b) Füttern mit Medium, das für das Wachstum und die Entwicklung der ausgesäten Zellen und/oder Gewebe günstig ist;
(c) Unterhalten der ausgesäten Zellen innerhalb des Konstrukts für einen ausreichenden Zeitraum, um das Gewebewachstum zu ermöglichen.

30. Verfahren gemäß Anspruch 29, das außerdem das Unterhalten der ausgesäten Zellen und/oder Gewebe innerhalb des Konstrukts unter Bedingungen, die das dreidimensionale Gewebewachstum fördern, umfasst.

31. Verfahren nach Anspruch 29 oder 30, wobei die ausgesäten Zellen und/oder Gewebe Zellen und/oder Gewebe von Binde-, Epithel-, Muskel-, Nerven- oder Drüsengewebe umfassen, oder Zellen und/oder Gewebe von vaskulären oder nicht-vaskulären Organen, oder irgendeine Kombination davon, aufweisen.

32. Verfahren zur Generierung differenzierter Zellen und/oder Gewebe aus dem Konstrukt gemäß einem der Ansprüche 1 bis 12, das irgendeines umfasst von:

(a) Besäen des Konstrukts mit Zellen und/oder Gewebe;

(b) Kontaktieren des besäten Konstrukts mit wenigstens einem Wachstumsfaktor, wie etwa basischem Fibroblasten-Wachstumsfaktor (bFGF), epidermalem Wachstumsfaktor (EGF), vom Gehirn abgeleitetem Nervenwachstumsfaktor (BDNF);

(c) Kontaktieren des besäten Konstrukts mit differenzierten Zellen;

(d) Kontaktieren des besäten Konstrukts mit Gewebe;

(e) Zugeben von Wachstumsmedium mit oder ohne Serum, wie etwa fötalem Humanserum (FHS), zu dem besäten Konstrukt;

(f) Platzieren des besäten Konstrukts in einem gerichteten elektromagnetischen Feld;

(g) Platzieren des besäten Konstrukts in einer vorgeformten Gussform mit einer bestimmten Hohlform, wie etwa einer ballonförmigen oder scheibenförmigen Gussform;

(h) Platzieren des besäten Konstrukts in einer Kulturumgebung mit simulierter nahezu null Schwerkraft;

(i) Kontaktieren des Konstrukts mit einer Probe von differenzierten Zellen;

(j) Versehen des besäten Konstrukts mit geeigneten Wachstumsbedingungen, einschließlich mechanischer Bedingungen, wie etwa Belastung oder Dehnung, chemischer Bedingungen, wie etwa ergänzende Nährstoffe oder Wachstumsfaktoren, und Umweltbedingungen, wie etwa veränderte Temperatur, pH-Wert oder atmosphärischer Druck, um die Differenzierung und Geweberegeneration zu fördern; oder

(k) Bewahren des besäten Konstrukts in einem lebensfähigen Zustand, so dass das Gewebe regeneriert wird.

33. Verfahren zur Generierung eines lebensfähigen neuronalen Gewebes von Vertebraten in vitro, das umfasst:

(a) Besäen des Konstrukts gemäß einem der Ansprüche 1 bis 12 mit primären neuralen Zellen oder neuronalem Gewebe von Vertebraten; und

(b) Behalten des besäten Konstrukts in Kultur unter Bedingungen, bei denen das lebensfähige neuronale Gewebe von Vertebraten generiert wird.

34. Verfahren gemäß Anspruch 33, wobei das Konstrukt mit neuralen Progenitorzellen, neuralen Stammzellen oder Neuroblastomen von Vertebraten, vorzugsweise Gliazellen von Vertebraten, besät wird.

35. Verfahren nach Anspruch 34, wobei das neuronale Gewebe von Vertebraten Säuger-Nervengewebe, vorzugsweise menschliches Nervengewebe, ist.


**Revendications**

1. Construction tridimensionnelle vivante comprenant des cellules fibroblastes et une matrice extracellulaire synthétisée *in vitro* par lesdites cellules fibroblastes, dans laquelle ladite construction peut être obtenue par un procédé comprenant :

l'incubation des cellules fibroblastes pendant 3 semaines dans le milieu défini chimiquement suivant :

(a) un rapport de 3 : 1 de DMEM à teneur élevée en glucose (4,5 g/L) ; avec de la L-glutamine et du pyruvate de sodium et le milieu F12 de Ham et l'ajout de :

(b) 5 $\mu$g/mL d'insuline ;

(c) 5 ng/mL d'acide sélénieux ;

(d) $10^{-4}$ M d'éthanolamine ;

(e) 150 $\mu$g/mL de sel de phosphate de magnésium d'acide L-ascorbique n-hydrate ;

(f) 2,5 ng/mL de facteur de croissance épidermique dans 5 $\mu$g/mL d'albumine de sérum humain ;

(g) 5 ng/mL de facteur de croissance des fibroblastes basique ;

(h) 1,0 x $10^{-7}$ M de dextraméthasone ;

(i) 2 x $10^{-10}$ M de L-3,3',5-triiodothyronine ;

(j) 4 x $10^{-3}$ M de Glutamax™ ;

(k) 1 $\mu$g/mL de glutathion ; et

(l) 1 % de pénicilline/streptomycine/amphotéricine B.

2. Construction selon la revendication 1, ayant un effort de rupture d'au moins 0,2.

3. Construction selon la revendication 1 ou la revendication 2, dans laquelle l'épaisseur est d'au moins 100 $\mu$m.

**4.** Construction selon l'une quelconque des revendications 1 à 3, dans laquelle la construction comprend en outre des cellules supplémentaires, un tissu supplémentaire ou toute combinaison de ceux-ci.

**5.** Construction selon la revendication 4, dans laquelle lesdites cellules supplémentaires ou ledit tissu supplémentaire sont d'un type autre que lesdites cellules fibroblastes.

**6.** Construction selon l'une quelconque des revendications 1 à 5, qui favorise l'une quelconque de la croissance, la prolifération, la migration et/ou l'acquisition de cellules de morphologie de type *in vivo* ou toute combinaison de celles-ci.

**7.** Construction selon la revendication 6, dans laquelle lesdites croissances, prolifération, migration et/ou acquisition de cellules de morphologie de type *in vivo* ou toute combinaison de celles-ci peuvent avoir lieu *in vivo* ou *in vitro.*

**8.** Construction selon la revendication 4, dans laquelle lesdites cellules supplémentaires, ledit tissu supplémentaire ou toute combinaison de ceux-ci peuvent comprendre des cellules souches, des cellules progénitrices, des cellules précurseurs ; des cellules ou un tissu d'origine conjonctive, épithéliale, musculaire, nerveuse ou glandulaire ; et des cellules provenant d'organes vasculaires et/ou non vasculaires comprenant les neuroblastomes, les myoblastes, les astrocytes, les cardiomyocytes, les myoblastes du muscle squelettique, les hépatocytes, les chondrocytes, les ostéoblastes, les fibroblastes, les kératinocytes, les cellules de Schwann, les cellules nerveuses, les cellules gliales, les cellules épithéliales, les cellules endothéliales, les cellules du muscle lisse, les cellules du muscle squelettique, les cellules du muscle cardiaque, les cellules stromales, les cellules mésangiales, les cellules mésenchymateuses, les cellules hématopoïétiques, les cellules dendritiques, les cellules du système immunitaire, le tissu nerveux, le tissu hépatique, le tissu aortique, le tissu veineux, le tissu capillaire, le cartilage, l'os, le muscle, les glandes ou les follicules pileux.

**9.** Construction selon la revendication 8, dans laquelle ladite construction supporte la croissance de cellules, de tissu, d'organes, ou toute combinaison de ceux-ci.

**10.** Construction selon la revendication 4, dans laquelle ladite construction confère un support nutritionnel, un support de développement, ou leur combinaison.

**11.** Construction selon la revendication 4, dans laquelle ladite construction confère un support mécanique et structurel.

**12.** Construction selon la revendication 4, dans laquelle ladite construction comprend des cellules neuronales.

**13.** Procédé d'utilisation de la construction telle que définie dans l'une quelconque des revendications 1 à 12, comprenant la modélisation d'un système cellulaire ou d'un système tissulaire biologique humain ou d'une combinaison de ceux-ci *in vitro.*

**14.** Procédé selon la revendication 13, dans lequel lesdites cellules sont alimentées en milieux asériques.

**15.** Procédé selon la revendication 14, dans lequel lesdits milieux asériques comprennent :

(a) des milieux de base
(b) un facteur de croissance des fibroblastes basique (bFGF) ou un facteur de croissance équivalent ;
(c) de l'insuline ;
(d) un glucocorticoïde ; et
(e) du sélénium ;

à des concentrations appropriées pour entretenir la croissance des fibroblastes.

**16.** Procédé selon la revendication 15, dans lequel lesdits milieux asériques comprennent en outre un ou plusieurs parmi :

(a) de la L-glutamine supplémentaire (de préférence sous la forme de Glutamax™) ;
(b) un facteur de croissance supplémentaire (un facteur de croissance épidermique (EGF), un facteur de croissance dérivé des plaquettes (PDGF), etc.)
(c) de la L-3,3',5-triiodothyronine ;
(d) un précurseur lipidique comprenant de l'éthanolamine ou équivalent, à savoir l'o-phosphoryléthanolamine ;

l'acide ascorbique (de préférence sous la forme stable d'un sel de phosphate magnésium d'acide L-ascorbique n-hydrate) ;
(e) l'acide ascorbique ;
(f) un antioxydant (tel que le glutathion (réduit) ou le Trolox™) ;
(g) un ou des acides aminés spécifiques supplémentaires ;
(h) tout autre nutriment ou facteur qui est compatible avec la croissance de cellules ; et
(i) tout autre nutriment ou facteur qui est compatible avec la production d'une matrice extracellulaire.

**17.** Procédé selon la revendication 16, dans lequel lesdits milieux exempts de sérum comprennent en outre tout autre nutriment ou facteur qui est compatible avec la croissance de cellules et la sécrétion de facteurs de croissance et/ou d'autres composés qui favorisent la croissance et le développement des cellules ou du tissu d'intérêt ensemencés.

**18.** Procédé selon la revendication 17, dans lequel lesdits milieux comprennent :

(a) un rapport de 3 : 1 de DMEM (à teneur élevée en glucose avec de la L-glutamine et du pyruvate de sodium) : F12 ou un rapport de 2 : 1 de IMEM : F12 ;
(b) 4 mM de Glutamax™;
(c) 2,5 ng/mL d'EGF dans de l'albumine de sérum humain ;
(d) 5,0 ng/mL de bFGF ;
(e) 90 $\mu$M d'insuline ;
(f) 100 nM de dexaméthasone ;
(g) 200 pM de L-3,3',5-triiodothyronine ;
(h) 100 $\mu$M d'éthanolamine ;
(i) 40 nM d'acide sélénieux ;
(j) 320 $\mu$M de sel de phosphate de magnésium d'acide L-ascorbique n-hydrate ;
(l) 1 % de pén/strep/amphB.

**19.** Procédé selon l'une quelconque des revendications 13 à 18, dans lequel la construction est ensemencée de cellules supplémentaires, de tissu supplémentaire ou de leur combinaison.

**20.** Procédé selon la revendication 19, dans lequel lesdites cellules supplémentaires, ledit tissu supplémentaire ou leur combinaison comprennent des cellules souches, des cellules progénitrices, des cellules précurseurs ; des cellules ou un tissu d'origine conjonctive, épithéliale, musculaire, nerveuse et/ou glandulaire ; et des cellules provenant d'organes vasculaires et/ou non vasculaires telles que des neuroblastomes, des myoblastes, des astrocytes, des cardiomyocytes, des myoblastes du muscle squelettique, des hépatocytes, des chondrocytes, des ostéoblastes, des fibroblastes, des kératinocytes, des cellules de Schwann, des cellules nerveuses, des cellules gliales, des cellules épithéliales, des cellules endothéliales, des cellules du muscle lisse, des cellules du muscle squelettique, des cellules du muscle cardiaque, des cellules stromales, des cellules mésangiales, des cellules mésenchymateuses, des cellules hématopoïétiques, des cellules dendritiques, des cellules du système immunitaire, un tissu nerveux, un tissu hépatique, un tissu aortique, un tissu veineux, un tissu capillaire, le cartilage, l'os, le muscle, les glandes ou les follicules pileux.

**21.** Procédé selon la revendication 19 ou 20, dans lequel lesdites cellules supplémentaires ou ledit tissu supplémentaire ou leur combinaison comprennent plus d'un type de cellules supplémentaires.

**22.** Procédé selon l'une quelconque des revendications 19 à 21, dans lequel lesdites cellules supplémentaires ou ledit tissu supplémentaire ou leur combinaison comprennent des neuroblastomes et des myoblastes.

**23.** Procédé selon l'une quelconque des revendications 19 à 22, dans lequel lesdites cellules supplémentaires proviennent d'un individu particulier et ledit système biologique qui est modélisé est propre audit individu particulier.

**24.** Procédé selon l'une quelconque des revendications 19 à 23, dans lequel lesdites cellules supplémentaires ou ledit tissu supplémentaire ou leur combinaison proviennent d'une source biologique particulière et ledit système biologique qui est modélisé est propre à ladite source biologique particulière.

**25.** Procédé selon la revendication 24, dans lequel ladite source biologique particulière comprend une source d'origine conjonctive, épithéliale, musculaire, nerveuse, circulatoire, comprenant une origine hormonale, immunitaire, pro-

EP 1 727 893 B1

venant d'organes vasculaires ou non vasculaires.

**26.** Procédé selon la revendication 24 ou 25, dans lequel ladite source biologique particulière comprend en outre un agent microbien choisi dans le groupe constitué par les agents viraux, prions, bactériens, fongiques et parasites.

**27.** Procédé selon la revendication 26, dans lequel ladite source biologique particulière est des cellules hépatocytes ou un tissu hépatique et ledit agent microbien est un virus de l'hépatite.

**28.** Procédé selon la revendication 27, comprenant en outre l'évaluation de l'effet d'un ou plusieurs agents supplémentaires sur ledit système biologique qui est modélisé, dans lequel lesdits un ou plusieurs agents comprennent des agents pharmaceutiques, des enzymes, des hormones, des petites molécules, des peptides, des polypeptides, des produits naturels, des extraits de produits naturels, des sels inorganiques, d'autres cellules, des facteurs de croissance, des facteurs de coagulation, des toxines, des poisons, des acides nucléiques, des inducteurs de contrainte mécanique, des générateurs de courant électrique, des générateurs de champ et d'impulsion électromagnétiques et/ou des inducteurs d'ondes sonores.

**29.** Procédé d'utilisation de la construction telle que définie dans l'une quelconque des revendications 1 à 12 pour la croissance de tissu comprenant les étapes :

(a) d'ensemencement de ladite construction avec des cellules supplémentaires et/ou du tissu supplémentaire ;
(b) d'alimentation en milieux favorables pour la croissance et le développement desdites cellules et/ou dudit tissu ensemencés ;
(c) de maintien desdites cellules ensemencées dans la construction pendant une période suffisante pour permettre la croissance de tissu.

**30.** Procédé selon la revendication 29, comprenant en outre le maintien desdites cellules et/ou dudit tissu ensemencés dans ladite construction dans des conditions qui favorisent la croissance de tissu tridimensionnel.

**31.** Procédé selon la revendication 29 ou 30, dans lequel lesdites cellules et/ou ledit tissu ensemencés comprennent des cellules et/ou un tissu de tissu conjonctif, épithélial, musculaire, nerveux ou glandulaire, ou comprennent des cellules et/ou un tissu d'organes vasculaires ou non vasculaires, ou toute combinaison de ceux-ci.

**32.** Procédé de production de cellules et/ou d'un tissu différenciés à partir de la construction selon l'une quelconque des revendications 1 à 12, comprenant l'un quelconque de :

(a) l'ensemencement de ladite construction avec des cellules et/ou du tissu ;
(b) la mise en contact de ladite construction ensemencée avec au moins un facteur de croissance tel qu'un facteur de croissance des fibroblastes basique (bFGF), un facteur de croissance de l'épiderme (EGF), un facteur de croissance des nerfs dérivés du cerveau (BDNF) ;
(c) la mise en contact de ladite construction ensemencée avec des cellules différenciées ;
(d) la mise en contact de ladite construction ensemencée avec un tissu ;
(e) l'ajout d'un milieu de croissance avec ou sans sérum tel qu'un sérum humain foetal (FHS) à ladite construction ensemencée ;
(f) le placement de ladite construction ensemencée dans un champ électromagnétique directionnel ;
(g) le placement de ladite construction ensemencée dans un moule préformé ayant une forme creuse particulière, tel qu'un moule en forme de ballon ou en forme de disque ;
(h) le placement de ladite construction ensemencée dans un environnement de culture à gravité proche de zéro simulé ;
(i) la mise en contact de ladite construction avec un échantillon de cellules différenciées ;
(j) la fourniture à ladite construction ensemencée de conditions de croissance appropriées comprenant des conditions mécaniques telles que la contrainte ou l'effort, des conditions chimiques telles que des nutriments de complément ou des facteurs de croissance, et des conditions environnementales telles qu'une température, un pH ou une pression atmosphérique modifié pour favoriser la différenciation et la régénération de tissu ; ou
(k) le maintien de ladite construction ensemencée dans une condition viable de sorte que le tissu est régénéré.

**33.** Procédé de génération de tissu neuronal de vertébré viable *in vitro* comprenant :

(a) l'ensemencement de la construction selon l'une quelconque des revendications 1 à 12 avec des cellules

36

nerveuses primaires ou un tissu neuronal de vertébré ; et

(b) le maintien de ladite construction ensemencée en culture dans des conditions où ledit tissu neuronal de vertébré viable est généré.

34. Procédé selon la revendication 33, dans lequel ladite construction est ensemencée avec des cellules progénitrices nerveuses, des cellules souches nerveuses, ou des neuroblastomes de vertébré, de préférence des cellules gliales de vertébré.

35. Procédé selon la revendication 34, dans lequel ledit tissu neuronal de vertébré est un tissu nerveux mammalien, de préférence un tissu nerveux humain.

*FIG. 1*

*FIG. 2*

*FIG. 3*

100 µm

20 µm

14 µm

10 µm

62 µm

FIG. 4

*FIG. 5*

15 µm

10 µm

6 µm    4 µm

230 µm

100 µm

18 μm

11 μm

100 μm

22 μm

216 μm

*FIG. 6*

**FIG. 7**

# Control - No Primary Antibody

*FIG. 8*

81 µm

100 µm

EP 1 727 893 B1

## Control - No Primary Antibody

*FIG. 9*

132 µm

100 µm

EP 1 727 893 B1

*FIG. 11*

9 µm

11 µm

10 µm

10 µm

9 µm

7 µm

10 µm

13 µm

*Control - Human Brain*

100 µm

FIG. 12

Red: Muscle

FIG. 13

5 µm

11 µm  6 µm

7 µm  6 µm  9 µm

8 µm

9 µm

116 µm

100 µm

*FIG. 14*

*FIG. 15A*

FG

230 μm

100 μm

**FIG. 15B**

LTM

110 μm

100 μm

**FIG. 15C**

**FIG. 15D**

**FIG. 15E**

**FIG. 16A**

**FIG. 16B**

**FIG. 16C**

**FIG. 16D**

**FIG. 16E**

**FIG. 16F**

**FIG. 16G**

**FIG. 16H**

**FIG. 16I**

**FIG. 16J**

**FIG. 16K**

**FIG. 16L**

**FIG. 17A**

**FIG. 17B**

**FIG. 17C**

**FIG. 17D**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 0040005297 A, P. R. Connelly
- WO 0029553 A
- EP 1131410 A
- US 93067304 A
- US 49914203 P

### Non-patent literature cited in the description

- **MARUSICH, M.F. et al.** *J. Neurobiol.,* 1994, vol. 25, 143-155
- **MISTRY et al.** *Proc Natl Acad Sci USA,* 2002, vol. 99 (3), 1621-6
- **SEGEV et al.** *Phys Rev Lett,* 2003, vol. 90 (16), 168101
- **MARUSICH et al.** *J. Neurobiol,* 1994, vol. 25 (2), 143-155
- **JIN et al.** *Exp Neurol,* 2002, vol. 177 (1), 265-75
- **TUSZYNSKI et al.** *J Comp Neurol,* 2002, vol. 449 (1), 88-101
- **HUANG et al.** *Annal Biomed Eng,* 1993, vol. 21 (3), 289-305
- **UYSAL et al.** *Plast Reconstr Surg,* 2003, vol. 112 (2), 540-6